# EUROPEAN PATENT APPLICATION

(11) **EP 4 545 634 A1**
(43) Date of publication of application: **30.04.2025**
(21) Application number: 23838769.0
(22) Date of filing: 30.06.2023
(51) Int. Cl.: C12N 7/01, C07K 7/04, C07K 14/145, A61K 35/766, A61P 35/00

(54) **RECOMBINANT ONCOLYTIC VIRUS AND USE THEREOF**

(30) Priority: 14.07.2022 CN 202210838778
(71) Applicant: Joint Biosciences (SH) Ltd., Pudong New Area Shanghai 201318 (CN)
(72) Inventor: ZHOU, Guoqing, Shanghai 201318 (CN); ZHANG, Fan, Shanghai 201318 (CN); MA, Liang, Shanghai 201318 (CN); TIAN, Ting, Shanghai 201318 (CN)
(74) Representative: Ullrich & Naumann PartG mbB
(86) International application number: PCT/CN2023/105011
(87) International publication number: WO 2024/012277

(57) **Abstract**

Provided are a recombinant oncolytic virus and a use thereof. The recombinant oncolytic virus includes an M protein and a cytokine encoded by an exogenous gene, wherein the M protein includes the following site mutations compared to an amino acid sequence as shown in SEQ ID NO 1: mutating of methionine at position 51 into arginine (M51R); mutating of valine at position 221 into phenylalanine (V221F); and mutating of serine at position 226 into arginine (S226R). The recombinant oncolytic viruses of the present application all have better infective ability and killing ability in vitro to abnormally proliferative (tumor) LLC cell, 4T1 cell, MC38 cell and Hela cell, and are not easy to eliminate in LLC cell, 4T1 cell, MC38 cell and Hela cell, greatly reducing the infective ability of the recombinant oncolytic viruses to normal cells.

## Description

### TECHNICAL FIELD

The present application relates to a technical field of biomedicine and, in particular, to a recombinant oncolytic virus and a use thereof.

### BACKGROUND ART

The oncolytic virus is a tumor-killing virus with an ability to replicate and has been accepted by general public as an important branch of tumor immunotherapy. The oncolytic virus can specifically target and infect a tumor cell, for example, by exploiting an inactivation or a defect of a tumor suppressor gene in the tumor cell to selectively infect the tumor cell; after infecting the tumor cell, the oncolytic virus will replicate in large numbers within the tumor cell and eventually destroy the tumor cell, thereby killing the tumor cell. At the same time, the oncolytic virus can futther provide an immune stimulation signal necessary to improve a host's own anti-cancer response, thereby attracting more immune cells to continue killing a residual tumor cell.

Although the oncolytic virus has a good application prospect in tumor immunotherapy, a wild-type oncolytic virus often causes an inflammation of a body's nervous system and other problems. A large pathogenic risk exists are in a process of using the wild-type virus to infect the tumor cell. Therefore, in order to further promote a clinical application of the oncolytic virus, the wild-type oncolytic virus needs to be engineered to obtain an attenuated oncolytic virus. The clinical application of the attenuated oncolytic virus can reduce a risk of the oncolytic virus and improve a safety of the oncolytic virus.

However, in a process of engineering the oncolytic virus, if only the wild-type oncolytic virus is randomly genetically engineered, although a toxicity of the wild-type oncolytic virus can be reduced, an engineered oncolytic virus may have a poor cure rate, or even the engineered oncolytic virus cannot be packaged, which is not conducive to promoting the clinical application of the oncolytic virus.

### SUMMARY

In order to further improve the in vitro and in vivo infectivity and killing ability of oncolytic viruses on tumor cells, and reduce the infectivity of oncolytic viruses on normal cells, the present application provides a recombinant oncolytic virus and a use thereof.

The recombinant oncolytic virus provided by the present disclosure adopts the following technical solutions.

A recombinant oncolytic virus, including an M protein and a cytokine encoded by an exogenous gene, wherein the M protein comprises the following site mutations compared to an amino acid sequence as shown in SEQ ID NO 1: mutating of methionine at position 51 into arginine (M51R); mutating of valine at position 221 into phenylalanine (V221F); and mutating of serine at position 226 into arginine (S226R).

Further, the M protein further includes one or more of the following site mutations: mutating of asparagine at position 32 into serine (N32S); and/or mutating of asparagine at position 49 into aspartic acid (N49D); and/or mutating of histidine at position 54 into tyrosine (H54Y); and/or mutating of valine at position 225 into isoleucine (V225I).

Further, the M protein further includes one or more of the following site mutations: knocking out of leucine at position 111; or, mutating of leucine at position 111 into alanine (L111A).

Further, the M protein further includes one or more of the following site mutations: mutating of glycine at position 21 into alanine (G21E); and/or mutating of methionine at position 33 into alanine (M33A); and/or mutating of alanine at position 133 into threonine (A133T).

In a specific embodiment, the site mutation of the M protein includes mutating of methionine at position 51 into arginine (M51R).

In a specific embodiment, the site mutation of the M protein includes mutating of valine at position 221 into phenylalanine (V221F).

In a specific embodiment, the site mutation of the M protein includes mutating of serine at position 226 into arginine (S226R).

In a specific embodiment, the site mutation of the M protein includes mutating of asparagine at position 32 into serine (N32S).

In a specific embodiment, the site mutation of the M protein includes mutating of asparagine at position 49 into aspartic acid (N49D).

In a specific embodiment, the site mutation of the M protein includes mutating of histidine at position 54 into tyrosine (H54Y).

In a specific embodiment, the site mutation of the M protein includes mutating of valine at position 225 into isoleucine (V225I).

In a specific embodiment, the site mutation of the M protein includes knocking out of leucine at position 111.

In a specific embodiment, the site mutation of the M protein includes mutating of leucine at position 111 into alanine (L111A).

In a specific embodiment, the site mutation of the M protein includes mutating of glycine at position 21 into alanine (G21E).

In a specific embodiment, the site mutation of the M protein includes mutating of methionine at position 33 into alanine (M33A).

In a specific embodiment, the site mutation of the M protein includes mutating of alanine at position 133 into threonine (A133T).

In a specific embodiment, the M protein has amino acid substitutions of M51R, V221F and S226R.

In a specific embodiment, the M protein has amino acid substitutions of N32S, N49D, M51R, H54Y, V221F, V225I and S226R.

In a specific embodiment, the M protein has amino acid substitutions of N32S, N49D, M51R, H54Y, knocking out of leucine at position 111, V221F, V225I and S226R.

In a specific embodiment, the M protein has amino acid substitutions of N32S, N49D, M51R, H54Y, L111A, V221F, V225I and S226R.

In a specific embodiment, the M protein has amino acid substitutions of G21E, N32S, N49D, M51R, H54Y, V221F, V225I and S226R.

In a specific embodiment, the M protein has amino acid substitutions of G21E, N32S, M33A, N49D, M51R, H54Y, V221F, V225I and S226R.

In a specific embodiment, the M protein has amino acid substitutions of G21E, N32S, M33A, N49D, M51R, H54Y, A133T, V221F, V225I and S226R.

In a specific embodiment, the M protein has amino acid substitutions of N32S, M33A, N49D, M51R, H54Y, V221F, V225I and S226R.

In a specific embodiment, the M protein has amino acid substitutions of N32S, M33A, N49D, M51R, H54Y, A133T, V221F, V2251 and S226R.

In a specific embodiment, the M protein has amino acid substitutions of N32S, N49D, M51R, H54Y, A133T, V221F, V225I and S226R.

In the present application, an M protein in a wild-type Indiana MuddSummer subtype strain of the VSV includes an amino acid sequence as shown in SEQ ID NO 1.

In a specific embodiment, the M protein includes an amino acid sequence as shown in SEQ ID NO 2.

In a specific embodiment, the M protein includes an amino acid sequence as shown in SEQ ID NO 3.

In a specific embodiment, the M protein includes an amino acid sequence as shown in SEQ ID NO 4.

In a specific embodiment, the M protein includes an amino acid sequence as shown in SEQ ID NO 5.

In a specific embodiment, the M protein includes an amino acid sequence as shown in SEQ ID NO 6.

In a specific embodiment, the M protein includes an amino acid sequence as shown in SEQ ID NO 7.

In a specific embodiment, the M protein includes an amino acid sequence as shown in SEQ ID NO 8.

In a specific embodiment, the M protein includes an amino acid sequence as shown in SEQ ID NO 9.

In a specific embodiment, the M protein includes an amino acid sequence as shown in SEQ ID NO 10.

In a specific embodiment, the M protein includes an amino acid sequence as shown in SEQ ID NO 11.

Further, the cytokine is at least one selected from a group consisting of: interleukins (IL), interferons (IFN), tumor necrosis factors (TNF), colony stimulating factors (CSF), transforming growth factor-β family (TGF-β family), and chemokine family.

Further, the cytokine is at least one selected from a group consisting of: GM-CSF, G-CSF, M-CSF, IL-1, IL-2, IL-4, IL-5, IL-6, IL-9, IL-10, IL-12, IL-13, IL-15, IL-17, IL-18, IL-23, IL-27, IFN-a, IFN-β, IFN-γ, IFN-β, TGF-β and TNF-α.

Further, the cytokine is at least one selected from a group consisting of: GM-CSF, IL-2, IL-9, IL-12, IL-15, IL-18, TNF-α and IFN-β.

In a specific embodiment, the cytokine is GM-CSF.

In a specific embodiment, the cytokine is IL-2.

In a specific embodiment, the cytokine is IL-12.

In a specific embodiment, the cytokine is IL-15.

In a specific embodiment, the cytokine is IL-18.

In a specific embodiment, the cytokine is TNF-α.

In a specific embodiment, the cytokine is IFN-β.

In a specific embodiment, the cytokine GM-CSF includes an amino acid sequence as shown in SEQ ID NO 20.

In a specific embodiment, the cytokine IL-2 includes an amino acid sequence as shown in SEQ ID NO 21.

In a specific embodiment, the cytokine IL-12-A includes an amino acid sequence as shown in SEQ ID NO 22.

In a specific embodiment, the cytokine IL-12-B includes an amino acid sequence as shown in SEQ ID NO 23.

In a specific embodiment, the cytokine IL-15 includes an amino acid sequence as shown in SEQ ID NO 24.

In a specific embodiment, the cytokine IL-18 includes an amino acid sequence as shown in SEQ ID NO 25.

In a specific embodiment, the cytokine TNF-α includes an amino acid sequence as shown in SEQ ID NO 26.

In a specific embodiment, the cytokine IFN-β includes an amino acid sequence as shown in SEQ ID NO 27.

In a specific embodiment, the cytokine IL-12-A includes an amino acid sequence as shown in SEQ ID NO 28.

In a specific embodiment, the cytokine IL-12-A includes an amino acid sequence as shown in SEQ ID NO 29.

In a specific embodiment, the cytokine IL-12-A includes an amino acid sequence as shown in SEQ ID NO 30.

In a specific embodiment, the cytokine IL-15 includes an amino acid sequence as shown in SEQ ID NO 31.

In a specific embodiment, the cytokine IL-18 includes an amino acid sequence as shown in SEQ ID NO 32.

A recombinant oncolytic virus, including the M protein as described above; the recombinant oncolytic virus further includes a G protein, wherein the G protein includes one or more of the following site mutations compared to an amino acid sequence as shown in SEQ ID NO 12: mutating of valine at position 53 into isoleucine (V531); and/or, mutating of alanine at position 141 into valine (A141V); and/or, mutating of aspartic acid at position 172 into tyrosine (D172Y); and/or, mutating of lysine at position 217 into glutamic acid (K217E); and/or, mutating of aspartic acid at position 232 into glycine and/or, mutating of valine at position 331 to alanine (V331A); and/or, mutating of valine at position 371 into glutamic acid (V371E); and/or, mutating of glycine at position 436 into aspartic acid (G436D); and/or, mutating of threonine at position 438 into serine (T438S); and/or, mutating of phenylalanine at position 453 into leucine (F453L); and/or, mutating of threonine at position 471 into isoleucine (T471I); and/or, mutating of tyrosine at position 487 into histidine (Y487H).

In a specific embodiment, the site mutation of the G protein includes mutating of valine at position 53 into isoleucine (V53I).

In a specific embodiment, the site mutation of the G protein includes mutating of alanine at position 141 into valine (A141V).

In a specific embodiment, the site mutation of the G protein includes mutating of aspartic acid at position 172 into tyrosine (D172Y).

In a specific embodiment, the site mutation of the G protein includes mutating of lysine at position 217 into glutamic acid (K217E).

In a specific embodiment, the site mutation of the G protein includes mutating of aspartic acid at position 232 into glycine (D232G).

In a specific embodiment, the site mutation of the G protein includes mutating of valine at position 331 into alanine (V331A).

In a specific embodiment, the site mutation of the G protein includes mutating of valine at position 371 into glutamic acid (V371E).

In a specific embodiment, the site mutation of the G protein includes mutating of glycine at position 436 into aspartic acid (G436D).

In a specific embodiment, the site mutation of the G protein includes mutating of threonine at position 438 into serine (T438S).

In a specific embodiment, the site mutation of the G protein includes mutating of phenylalanine at position 453 into leucine (F453L).

In a specific embodiment, the site mutation of the G protein includes mutating of threonine at position 471 into isoleucine (T471I).

In a specific embodiment, the site mutation of the G protein includes mutating of tyrosine at position 487 into histidine (Y487H).

In a specific embodiment, the G protein has an amino acid substitution of V53I.

In a specific embodiment, the G protein has amino acid substitutions of V53I and A141V.

In a specific embodiment, the G protein has amino acid substitutions of V53I, A141V and D172Y.

In a specific embodiment, the G protein has amino acid substitutions of V53I, A141V, D172Y and K217E.

In a specific embodiment, the G protein has amino acid substitutions of V53I, A141V, D172Y, K217E and D232G.

In a specific embodiment, the M protein has amino acid substitutions of V53I, A141V, D172Y, K217E, D232G and V331A.

In a specific embodiment, the G protein has amino acid substitutions of V53I, A141V, D172Y, K217E, D232G, V331A and V371E.

In a specific embodiment, the G protein has amino acid substitutions of V53I, A141V, D172Y, K217E, D232G, V331A, V371E and G436D.

In a specific embodiment, the G protein has amino acid substitutions of V53I, A141V, D172Y, K217E, D232G, V331A, V371E, G436D and T438S.

In a specific embodiment, the G protein has amino acid substitutions of V53I, A141V, D172Y, K217E, D232G, V331A, V371E, G436D, T438S and F453L.

In a specific embodiment, the G protein has amino acid substitutions of V53I, A141V, D172Y, K217E, D232G, V331A, V371E, G436D, T438S, F453L and T471I.

In a specific embodiment, the G protein has amino acid substitutions of V53I, A141V, D172Y, K217E, D232G, V331A, V371E, G436D, T438S, F453L, T471I and Y487H.

In a specific embodiment, the G protein has amino acid substitutions of A141V, D172Y, K217E, D232G, V331A, V371E, G436D, T438S, F453L, T471I and Y487H.

In a specific embodiment, the G protein has amino acid substitutions of D172Y, K217E, D232G, V331A, V371E, G436D, T438S, F453L, T471I and Y487H.

In a specific embodiment, the G protein has amino acid substitutions of K217E, D232G, V331A, V371E, G436D, T438S, F453L, T471I and Y487H.

In a specific embodiment, the G protein has amino acid substitutions of D232G, V331A, V371E, G436D, T438S, F453L, T471I and Y487H.

In a specific embodiment, the G protein has amino acid substitutions of V331A, V371E, G436D, T438S, F453L, T471I and Y487H.

In a specific embodiment, the G protein has amino acid substitutions of V371E, G436D, T438S, F453L, T471I and Y487H.

In a specific embodiment, the G protein has amino acid substitutions of G436D, T438S, F453L, T471I and Y487H.

In a specific embodiment, the G protein has amino acid substitutions of T438S, F453L, T471I and Y487H.

In a specific embodiment, the G protein has amino acid substitutions of F453L, T471I and Y487H.

In a specific embodiment, the G protein has amino acid substitutions of T471I and Y487H.

In a specific embodiment, the G protein has an amino acid substitution of Y487H.

In the present application, a G protein in a wild-type Indiana MuddSummer subtype strain of the VSV includes an amino acid sequence as shown in SEQ ID NO 12.

In a specific embodiment, the G protein includes an amino acid sequence as shown in SEQ ID NO 13.

A recombinant oncolytic virus, including the M protein as described above or the M protein and G protein as described above; the recombinant oncolytic virus further includes an N protein, wherein the N protein includes one or more of the following site mutations compared to an amino acid sequence as shown in SEQ ID NO 14: mutating of isoleucine at position 14 into valine (I14V); and/or, mutating of arginine at position 155 into lysine (R155K); and/or, mutating of serine at position 353 into asparagine (S353N).

In a specific embodiment, the site mutation of the N protein includes mutating of isoleucine at position 14 into valine (I14V).

In a specific embodiment, the site mutation of the N protein includes mutating of arginine at position 155 into lysine (R155K).

In a specific embodiment, the site mutation of the N protein includes mutating of serine at position 353 into asparagine (S353N).

In a specific embodiment, the N protein has an amino acid substitution of 114V.

In a specific embodiment, the N protein has amino acid substitutions of I14V and R155K.

In a specific embodiment, the N protein has amino acid substitutions of I14V, R155K and S353N.

In a specific embodiment, the N protein has amino acid substitutions of R155K and S353N.

In a specific embodiment, the N protein has an amino acid substitution of S353N.

In the present application, an N protein in a wild-type Indiana MuddSummer subtype strain of the VSV includes an amino acid sequence as shown in SEQ ID NO 14.

In a specific embodiment, the N protein includes an amino acid sequence as shown in SEQ ID NO 15.

A recombinant oncolytic virus, including the M protein as described above; or the M protein and G protein as described above; or the M protein, G protein and N protein as described above; the recombinant oncolytic virus further includes a P protein, wherein the P protein includes one or more of the following site mutations compared to an amino acid sequence as shown in SEQ ID NO 16: mutating of arginine at position 50 into lysine (R50K); and/or mutating of valine at position 76 into alanine (V76A); and/or mutating of asparagine at position 99 into glutamic acid (D99E); and/or mutating of leucine at position 126 into serine (L126S); and/or mutating of leucine at position 140 into serine (L140S); and/or mutating of histidine at position 151 into tyrosine (H151Y); and/or mutating of isoleucine at position 168 into methionine (T168M); and/or mutating of lysine at position 170 into glutamic acid (K170E); and/or mutating of tyrosine at position 189 into serine (Y189S); and/or mutating of asparagine at position 237 into aspartic acid (N237D).

In a specific embodiment, the site mutation of the P protein includes mutating of arginine at position 50 into lysine (R50K).

In a specific embodiment, the site mutation of the P protein includes mutating of valine at position 76 into alanine (V76A).

In a specific embodiment, the site mutation of the P protein includes mutating of asparagine at position 99 into glutamic acid (D99E).

In a specific embodiment, the site mutation of the P protein includes mutating of leucine at position 126 into serine (L126S).

In a specific embodiment, the site mutation of the P protein includes mutating of leucine at position 140 into serine (L140S).

In a specific embodiment, the site mutation of the P protein includes mutating of histidine at position 151 into tyrosine (H151Y).

In a specific embodiment, the site mutation of the P protein includes mutating of isoleucine at position 168 into methionine (I168M).

In a specific embodiment, the site mutation of the P protein includes mutating of lysine at position 170 into glutamic acid (K170E).

In a specific embodiment, the site mutation of the P protein includes mutating of tyrosine at position 189 into serine (Y189S).

In a specific embodiment, the site mutation of the P protein includes mutating of asparagine at position 237 into aspartic acid (N237D).

In a specific embodiment, the P protein has an amino acid substitution of R50K.

In a specific embodiment, the P protein has amino acid substitutions of R50K and V76A.

In a specific embodiment, the P protein has amino acid substitutions of R50K, V76A and D99E.

In a specific embodiment, the P protein has amino acid substitutions of R50K, V76A, D99E and L126S.

In a specific embodiment, the P protein has amino acid substitutions of R50K, V76A, D99E, L126S and L140S.

In a specific embodiment, the P protein has amino acid substitutions of R50K, V76A, D99E, L126S, L140S and H151Y.

In a specific embodiment, the P protein has amino acid substitutions of R50K, V76A, D99E, L126S, L140S, H151Y and I168M.

In a specific embodiment, the P protein has amino acid substitutions of R50K, V76A, D99E, L126S, L140S, H151Y, T168M and K170E.

In a specific embodiment, the P protein has amino acid substitutions of R50K, V76A, D99E, L126S, L140S, H151Y, I168M, K170E and Y189S.

In a specific embodiment, the P protein has amino acid substitutions of R50K, V76A, D99E, L126S, L140S, H151Y, I168M, K170E, Y189S and N237D.

In a specific embodiment, the P protein has amino acid substitutions of V76A, D99E, L126S, L140S, H151Y, I168M, K170E, Y189S and N237D.

In a specific embodiment, the P protein has amino acid substitutions of D99E, L126S, L140S, H151Y, I168M, K170E, Y189S and N237D.

In a specific embodiment, the P protein has amino acid substitutions of L126S, L140S, H151Y, I168M, K170E, Y189S and N237D.

In a specific embodiment, the P protein has amino acid substitutions of L140S, H151Y, I168M, K170E, Y189S and N237D.

In a specific embodiment, the P protein has amino acid substitutions of H151Y, I168M, K170E, Y189S and N237D.

In a specific embodiment, the P protein has amino acid substitutions of I168M, K170E, Y189S and N237D.

In a specific embodiment, the P protein has amino acid substitutions of K170E, Y189S and N237D.

In a specific embodiment, the P protein has amino acid substitutions of Y189S and N237D.

In a specific embodiment, the P protein has an amino acid substitution of N237D.

In the present application, a P protein in in a wild-type Indiana MuddSummer subtype strain of the VSV includes an amino acid sequence as shown in SEQ ID NO 16.

In a specific embodiment, the P protein includes an amino acid sequence as shown in SEQ ID NO 17.

A recombinant oncolytic virus, including the M protein as described above; or the M protein and G protein as described above; or the M protein, G protein and N protein as described above; or the M protein, G protein, N protein and P protein as described above; the recombinant oncolytic virus further includes an L protein, wherein the L protein includes one or more of the following site mutations compared to an amino acid sequence as shown in SEQ ID NO 18: mutating of serine at position 87 into proline (S87P); and/or, mutating of isoleucine at position 487 into threonine (I487T).

In a specific embodiment, the site mutation of the L protein includes mutating of serine at position 87 into proline (S87P).

In a specific embodiment, the site mutation of the L protein includes mutating of isoleucine at position 487 into threonine (I487T).

In a specific embodiment, the L protein has amino acid substitutions of S87P andI487T.

In the present application, an L protein in a wild-type Indiana MuddSummer subtype strain of the VSV includes an amino acid sequence as shown in SEQ ID NO 18.

In a specific embodiment, the L protein includes an amino acid sequence as shown in SEQ ID NO 19.

In some specific embodiments, the recombinant oncolytic virus is obtained by site-directed mutagenesis based on a rhabdovirus.

In some specific embodiments, the recombinant oncolytic virus is obtained by site-directed mutagenesis based on a vesicular stomatitis virus (abbreviated "VSV").

In some specific embodiments, the recombinant oncolytic virus is obtained by site-directed mutagenesis based on an Indiana MuddSummer subtype strain of the VSV.

In some specific embodiments, the recombinant oncolytic virus further includes or expresses an exogenous target protein.

In some specific embodiments, the recombinant oncolytic virus includes a nucleic acid molecule; the nucleic acid molecule includes a nucleic acid sequence encoding the M protein with the site mutation(s), and/or a nucleic acid sequence encoding the G protein with the site mutation(s), and/or a nucleic acid sequence encoding the N protein with the site mutation(s), and/or a nucleic acid sequence encoding the P protein with the site mutation(s), and/or a nucleic acid sequence encoding the L protein with the site mutation(s), and a nucleic acid sequence encoding the cytokine.

In some specific embodiments, in the nucleic acid molecule, the nucleic acid sequence encoding the cytokine is located between the nucleic acid sequence encoding the G protein with the site mutation(s) and the nucleic acid sequence encoding the L protein with the site mutation(s).

In some specific embodiments, in the nucleic acid molecule, the nucleic acid sequence encoding the cytokine is located between the nucleic acid sequence encoding the M protein with the site mutation(s), and/or the nucleic acid sequence encoding the G protein with the site mutation(s), and/or the nucleic acid sequence encoding the N protein with the site mutation(s), and/or the nucleic acid sequence encoding the P protein with the site mutation(s), and/or the nucleic acid sequence encoding the L protein with the site mutation(s).

In a second aspect, the present application provides an expression vector of the recombinant oncolytic virus and adopts the following technical solution.

An expression vector of the recombinant oncolytic virus capable of expressing the recombinant oncolytic viruses as described above.

In a third aspect, the present application provides a virus-producing cell and adopts the following technical solution.

A virus-producing cell capable of producing the recombinant oncolytic virus as described above.

In a fourth aspect, the present application provides a vaccine and adopts the following technical solution.

A vaccine prepared by using the recombinant oncolytic virus as described above.

In a fifth aspect, the present application provides a pharmaceutical composition and adopts the following technical solution.

A pharmaceutical composition, including the recombinant oncolytic virus or the vaccine as described above, and optionally a pharmaceutically acceptable carrier.

In a sixth aspect, the present application provides a method for preparing the recombinant oncolytic virus, the expression vector of the recombinant oncolytic virus, the virus-producing cell, the vaccine, or the pharmaceutical composition as described above.

In a seventh aspect, the present application provides a use of the recombinant oncolytic virus, the expression vector of the recombinant oncolytic virus, the virus-producing cell, the vaccine or the pharmaceutical composition in preparing a medicine for preventing and/or treating a disease and/or disorder.

In some specific embodiment, the recombinant oncolytic virus, the expression vector of the recombinant oncolytic virus, the virus-producing cell, the vaccine and/or the pharmaceutical composition are used in a method for sustained killing of an abnormally proliferative cell.

In some specific embodiments, the abnormally proliferative cell is selected from a group consisting of: a tumor cell and a cell associated with a tumor tissue.

In an eighth aspect, the present application provides a use of the recombinant oncolytic virus, the vaccine or the pharmaceutical composition as described above in preparing a medicine for treating a tumor.

In some specific embodiments, the tumor includes a solid tumor or a hematological tumor.

In some specific embodiments, the tumor includes but is not limited to acute lymphoblastic leukemia, acute B-cell leukemia, chronic non-lymphocytic leukemia, non-Hodgkin's lymphoma, anal cancer, astrocytoma, basal cell carcinoma, bile duct cancer, bladder cancer, mastocarcinoma, breast cancer, cervical cancer, chronic myeloproliferative tumors, colorectal cancer, endometrial cancer, ependymoma, esophageal cancer, diffuse large B-cell lymphoma, sensorineuroblastoma, Ewing's sarcoma, fallopian tube cancer, gallbladder cancer, gastric cancer, gastrointestinal carcinoid tumors, hepatocellular carcinoma, hypopharyngeal cancer, Kaposi sarcoma, kidney cancer, Langerhans cell hyperplasia, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumors, pharyngeal cancer, pituitary tumors, prostate cancer, rectal cancer, renal cell carcinoma, retinoblastoma, skin cancer, small cell lung cancer, small intestine cancer, squamous neck cancer, testicular cancer, thymoma, thyroid cancer, uterine cancer, vaginal cancer and vascular tumors.

In summary, the present application has the following beneficial effects:

The recombinant oncolytic viruses of the present application all have better infective ability to LLC cell, 4T1 cell, MC38 cell and Hela cell, and have better killing ability to LLC cell, 4T1 cell, MC38 cell and Hela cell in vitro, and are not easy to eliminate in LLC cell, 4T1 cell, MC38 cell and Hela cell. In addition, the recombinant oncolytic viruses of the present application have poor infective ability and have poor killing ability to a normal MEF cell in vitro and are easy to eliminate in normal MEF cell. In view of this, the recombinant oncolytic viruses of the present application can be well used for infecting and killing tumor, cancer and other cells, and are not easy to eliminate in tumor, cancer and other cells, further improving the cure rate of the recombinant oncolytic viruses for tumor, cancer and other cells. Moreover, the recombinant oncolytic viruses of the present application can not damage normal cells and are easier to eliminate in the normal cells, further ensuring the safety of the normal cells.

### BRIEF DESCRIPTION OF THE DRAWINGS.

FIG. 1 shows a test result of an infective ability of the recombinant oncolytic viruses prepared in Preparation Examples 1-70 of the present application and a wild-type oncolytic virus to LLC cell.
FIG. 2 shows a test result of an infective ability of the recombinant oncolytic viruses prepared in Preparation Examples 1-70 of the present application and a wild-type oncolytic virus to 4T1 cell.
FIG. 3 shows a test result of an infective ability of the recombinant oncolytic viruses prepared in Preparation Examples 1-70 of the present application and a wild-type oncolytic virus to MC38 cell.
FIG. 4 shows a test result of an infective ability of the recombinant oncolytic viruses prepared in Preparation Examples 1-70 of the present application and a wild-type oncolytic virus to Hela cell.
FIG. 5 shows a test result of an infective ability of the recombinant oncolytic viruses prepared in Preparation Examples 1-70 of the present application and a wild-type oncolytic virus to MEF cell.
FIG. 6 shows a test result of an infective ability of the recombinant oncolytic viruses prepared in Preparation Examples 71-105 of the present application and a wild-type oncolytic virus to LLC cell.
FIG. 7 shows a test result of an infective ability of the recombinant oncolytic viruses prepared in Preparation Examples 71-105 of the present application and a wild-type oncolytic virus to 4T1 cell.
FIG. 8 shows a test result of an infective ability of the recombinant oncolytic viruses prepared in Preparation Examples 71-105 of the present application and a wild-type oncolytic virus to MC38 cell.
FIG. 9 shows a test result of an infective ability of the recombinant oncolytic viruses prepared in Preparation Examples 71-105 of the present application and a wild-type oncolytic virus to Hela cell.
FIG. 10 shows a test result of an infective ability of the recombinant oncolytic viruses prepared in Preparation Examples 71-105 of the present application and a wild-type oncolytic virus to MEF cell.
FIG. 11 shows a test result of a killing ability of the recombinant oncolytic viruses prepared in Preparation Examples 1-70 of the present application and a wild-type oncolytic virus to LLC cell in vitro.
FIG. 12 shows a test result of a killing ability of the recombinant oncolytic viruses prepared in Preparation Examples 1-70 of the present application and a wild-type oncolytic virus to 4T1 cell in vitro.
FIG. 13 shows a test result of a killing ability of the recombinant oncolytic viruses prepared in Preparation Examples 1-70 of the present application and a wild-type oncolytic virus to MC38 cell in vitro.
FIG. 14 shows a test result of a killing ability of the recombinant oncolytic viruses prepared in Preparation Examples 1-70 of the present application and a wild-type oncolytic virus to Hela cell in vitro.
FIG. 15 shows a test result of a killing ability of the recombinant oncolytic viruses prepared in Preparation Examples 1-70 of the present application and a wild-type oncolytic virus to MEF cell in vitro.
FIG. 16 shows a test result of a killing ability of the recombinant oncolytic viruses prepared in Preparation Examples 71-105 of the present application and a wild-type oncolytic virus to LLC cell in vitro.
FIG. 17 shows a test result of a killing ability of the recombinant oncolytic viruses prepared in Preparation Examples 71-105 of the present application and a wild-type oncolytic virus to 4T1 cell in vitro.
FIG. 18 shows a test result of a killing ability of the recombinant oncolytic viruses prepared in Preparation Examples 71-105 of the present application and a wild-type oncolytic virus to MC38 cell in vitro.
FIG. 19 shows a test result of a killing ability of the recombinant oncolytic viruses prepared in Preparation Examples 71-105 of the present application and a wild-type oncolytic virus to Hela cell in vitro.
FIG. 20 shows a test result of a killing ability of the recombinant oncolytic viruses prepared in Preparation Examples 71-105 of the present application and a wild-type oncolytic virus to t MEF cell in vitro.
FIG. 21 shows an expression of IFN-β induced by the recombinant oncolytic viruses prepared in Preparation Examples 1-70 of the present application and a wild-type oncolytic virus in LLC cell.
FIG. 22 shows an expression of IFN-β induced by the recombinant oncolytic viruses prepared in Preparation Examples 1-70 of the present application and a wild-type oncolytic virus in 4T1 cell.
FIG. 23 shows an expression of IFN-β induced by the recombinant oncolytic viruses prepared in Preparation Examples 1-70 of the present application and a wild-type oncolytic virus in MC38 cell.
FIG. 24 shows an expression of IFN-β induced by the recombinant oncolytic viruses prepared in Preparation Examples 1-70 of the present application and a wild-type oncolytic virus in Hela cell.
FIG. 25 shows an expression of IFN-β induced by the recombinant oncolytic viruses prepared in Preparation Examples 1-70 of the present application and a wild-type oncolytic virus in MEF cell.
FIG. 26 shows an expression of IFN-β induced by the recombinant oncolytic viruses prepared in Preparation Examples 71-105 of the present application and a wild-type oncolytic virus in LLC cell.
FIG. 27 shows an expression of IFN-β induced by the recombinant oncolytic viruses prepared in Preparation Examples 71-105 of the present application and a wild-type oncolytic virus in 4T1 cell.
FIG. 28 shows an expression of IFN-β induced by the recombinant oncolytic viruses prepared in Preparation Examples 71-105 of the present application and a wild-type oncolytic virus in MC38 cell.
FIG. 29 shows an expression of IFN-β induced by the recombinant oncolytic viruses prepared in Preparation Examples 71-105 of the present application and a wild-type oncolytic virus in Hela cell.
FIG. 30 shows an expression of IFN-β induced by the recombinant oncolytic viruses prepared in Preparation Examples 71-105 of the present application and a wild-type oncolytic virus in MEF cell.

In the above figures, number 0 on the horizontal axis represents the wild-type oncolytic virus; numbers 1-105 on the horizontal axis represent the recombinant oncolytic viruses prepared in Preparation Examples 1-105, respectively.

Log₁₀ TCID50 on the vertical axis represents values of TCID50 calculated by a Karber method. The larger the value of Log₁₀ TCID50, the better the infective ability of a recombinant oncolytic virus to the cell; the smaller the value of Log₁₀ TCID50, the poorer the infective ability of the recombinant oncolytic virus to the cell;
OD₅₇₀ on the vertical axis represents the OD value of the cell. The larger the value of OD₅₇₀, the poorer the killing ability of a recombinant oncolytic virus to the cell; the smaller the value of OD₅₇₀, the better the killing ability of the recombinant oncolytic virus to the cell.

The IFN-β level on the vertical axis represents the expression level of the IFN-β gene. The larger the value of the IFN-β level, the weaker the reproduction ability of a recombinant oncolytic virus in the cell and the easier of the recombinant oncolytic virus to eliminate in the cell; the smaller the value of the IFN-β level, the stronger the reproduction ability of the recombinant oncolytic virus in the cell, and the more difficult of the recombinant oncolytic virus to eliminate in the cell.

Those skilled in the art can easily perceive other aspects and advantages of the present application from the following detailed description. Only exemplary embodiments of the present application are shown and described in the following detailed description. As those skilled in the art will appreciate, the teachings of the present application enable those skilled in the art to make modifications to the specific embodiments disclosed without departing from the spirit and scope of the invention to which the present application relates. Accordingly, the drawings and descriptions in the specification of the present application are merely illustrative and not restrictive.

### DETAILED DESCRIPTION

The implementation of the invention will be described below by means of specific embodiments. Those skilled in the art can easily understand other advantages and effects of the invention from the contents described herein.

### TERMS DEFINITION

The term "oncolytic virus", as used herein, generally refers to a virus that can replicate in tumor cells and kill tumor cells. Oncolytic viruses include, but are not limited to, vesicular stomatitis virus (VSV), poxvirus, herpes simplex virus, measles virus, Semliki Forest virus, poliovirus, reovirus, Seneca Valley virus, Echo-type enterovirus, coxsackievirus, Newcastle disease virus, and Maraba virus. In certain embodiments, the oncolytic virus is modified to improve the selectivity for tumor cells. In certain embodiments, the oncolytic virus is modified to reduce its immunogenicity.

In some embodiments, the oncolytic virus described herein is a VSV.

In some embodiments, the VSV is a mutant of the Indiana MuddSummer subtype strain of the VSV.

In some embodiments, the M protein, and/or the G protein, and/or the N protein, and/or the P protein, and/or the L protein of the VSV may be subjected to site-directed mutagenesis.

In certain embodiments, the recombinant oncolytic virus described herein may be an oncolytic virus that has been genetically modified, such as modified by modification of one or more genes, so as to improve its tumor selectivity and/or preferentially replicate in dividing cells. The "genetically modified" may refer to modification of genes involved in DNA/RNA replication, nucleic acid metabolism, host tropism, surface attachment, virulence, lysis and diffusion, or modification of integrating exogenous genes. The exogenous genes may include exogenous immune regulatory genes, exogenous screening genes and exogenous reporter genes. The oncolytic virus that has been modified may also be an oncolytic virus modified at an amino acid level, such as, by insertion, deletion, or substitution of one or more amino acids.

The term "M protein", as used herein, generally refers to a matrix protein of the VSV. The M protein is an important virulence factor of the VSV and is also a protein in the VSV that is known to interfere with the natural immune response of mice. The term "M protein" also encompasses homologs, orthologs, variants and functionally active fragments thereof. In the present application, the M protein in a wild-type Indiana MuddSummer subtype strain of the VSV may include an amino acid sequence as shown in SEQ ID NO 1. In the present application, the M protein of the oncolytic virus may include amino acid sequences as shown in SEQ ID NOs 2-11.

The term "G protein", as used herein, generally refers to a glycoprotein of the VSV, also known as the envelope protein. The term "G protein" also encompasses homologs, orthologs, variants and functionally active fragments thereof. In the present application, the G protein in a wild-type Indiana MuddSummer subtype strain of the VSV may include an amino acid sequence as shown in SEQ ID NO 12. In the present application, the G protein of the oncolytic virus may include an amino acid sequence as shown in SEQ ID NO 13.

The term "N protein", as used herein, generally refers to a nucleocapsid protein of the VSV. The term "N protein" also encompasses homologs, orthologs, variants and functionally active fragments thereof. In the present application, the N protein in Indiana MuddSummer subtype of a wild-type VSV may include an amino acid sequence as shown in SEQ ID NO 14. In the present application, the N protein of the oncolytic virus may include an amino acid sequence as shown in SEQ ID NO 15.

The term "P protein", as used herein, generally refers to a phosphoprotein of the VSV. The term "P protein" also encompasses homologs, orthologs, variants and functionally active fragments thereof. In the present application, the P protein in a wild-type Indiana MuddSummer subtype strain of the VSV may include an amino acid sequence as shown in SEQ ID NO 16. In the present application, the P protein of the oncolytic virus may include an amino acid sequence as shown in SEQ ID NO 17.

The term "L protein", as used herein, generally refers to an RNA poly E protein of the VSV. An L gene of the VSV encodes the RNA poly E protein. The term "L protein" also encompasses homologs, orthologs, variants and functionally active fragments thereof. In the present application, the L protein in a wild-type Indiana MuddSummer subtype strain of the VSV may include an amino acid sequence as shown in SEQ ID NO 18. In the present application, the L protein of the oncolytic virus may include an amino acid sequence as shown in SEQ ID NO 19.

In the present application, the mutation site of a protein is generally expressed as "amino acid + amino acid position + amino acid after mutation". In the present application, the mutation may include but is not limited to the addition, substitution absence and/or deletion of an amino acid. For example, the term "M51R" generally refers to the mutation of methionine M at position 51 to arginine R.

The term "amino acid substitution", as used herein, generally refers to the substitution of an amino acid residue present in a parent sequence with another amino acid residue. The amino acid in the parent sequence may be substituted, for example, via chemical synthesis or by recombinant methods known in the art. Therefore, "substitution at position xx" generally refers to the substitution of an amino acid present at position xx with an alternative amino acid residue. In the present application, the amino acid substitution may include an amino acid mutation.

In the present application, the term "mutation" generally refers to a change in the nucleotide or amino acid sequence of a wild-type molecule. Amino acid changes may include the substitution, deletion, absence, insertion, addition or truncation of an amino acid, or the processing or cleavage of a protein.

In the present application, the recombinant oncolytic virus means integration of an exogenous gene at the same time of an site-directed gene mutation on the M protein, and/or G protein, and/or N protein, and/or P protein, and/or L protein of the VSV. The exogenous gene specifically refers to a gene encoding a cytokine.

The term "cytokines", as used herein, refers to biologically active substances synthesized and secreted by immune cells (such as lymphocytes, monocytes and macrophages) and their related cells (such as vascular endothelial cells and fibroblasts) to regulate the functions of other immune cells or target cells. The cytokines belong to small molecule polypeptides or glycoproteins. Cytokines with immune regulation effects can be expressed by recombinant oncolytic viruses. According to their main functions, cytokines are divided into: interleukins (IL), interferons (IFN), tumor necrosis factors (TNF), colony stimulating factors (CSF), transforming growth factor-β family (TGF-β family), growth factors (GF), and chemokine family.

Interleukins include IL-1, IL-2, IL-7, IL-9, IL-15, IL-21, IL-4, IL-12 and IL-18.

Specifically, interleukin 1 (IL-1): IL-1 is a pleiotropic cytokine involved in cortical inflammatory response, cell growth and tissue repair. The IL-1 superfamily has 11 members, such as IL-1A, IL-1B, IL-1Ra, and IL-18. IL-1 is a drug target for some cancers and is also used in cell therapy. In terms of cellular immunotherapy, IL-1 can stimulate the proliferation of CD4+ T cells in vitro, induce the production of IL-2, co-stimulate the activation of CD8+/IL1R+ T cells, and stimulate the proliferation of mature B cell and the secretion of immunoglobulin.

Specifically, interleukin 2 (IL-2): IL-2, also known as T cell growth factor, is produced by T cells in response to antigens or mitotic stimulation and is widely used to promote the activation and proliferation of T cells and NK cells. IL-2 can stimulate proliferation of NK cells, increase cytotoxicity, and stimulate NK cells to secrete a variety of cytokines. However, further studies have found that IL-2 can cause overdifferentiation of T cells and induce apoptosis of activated T cells, and can also activate CD4+ FoxP3 Treg regulatory cells, thereby inhibiting activation of T cells and tumor killing activity. Therefore, IL-2 is considered to be a T cell regulatory factor rather than just an activation factor, so some studies have now used IL-7, IL-15, and IL-21 to replace IL-2.

Specifically, interleukin 7 (IL-7): IL-7 is a hematopoietic growth factor, secreted by stromal cells in the bone marrow and thymus, and shares the γc receptor subunit with IL-2 to stimulate the proliferation of lymphocyte progenitor cells. IL-7 can provide continuous stimulation signals for Naive T cells and memory T cells. As mentioned above, IL-7 will not activate CD4+ FoxP3+ Treg cells during the activation of CD8+ T cells. In clinical practice, IL-7 can also be used to restore the number of T cells after chemotherapy or hematopoietic stem cell transplantation. And IL-7 plays an important role in certain stages of maturation and can affect the proliferation of B cells. IL-7 can also act as a regulatory factor for intestinal mucosal lymphocytes.

Specifically, interleukin 15 (IL-15): IL-15 has a similar structure to IL-2, shares the γc receptor subunit, and belongs to the family with 4 α-helix bundles (others include, such as, IL-2, IL-4, IL-7, IL-9, G-CSF and GM-CSF). IL-15 regulates the activation and proliferation of T cells and NK cells. IL-15 mainly kills virus-infected cells in the innate immune system. Also, IL-15 can activate NKT cells and γδT cells. In immune cell therapy, IL-15 does not cause apoptosis of activated T cells, but activates CD8+ effector T cells. IL-15 maintains the survival of memory T cells, thus playing an important role in long-term anti-tumor activity.

Specifically, interleukin 21 (IL-21): IL-21 also belongs to the IL-2 family, shares the γc receptor subunit, has a strong regulatory effect on the cells of the immune system, and can induce cell division and proliferation in its target cells. In cellular immunotherapy, IL-21 can promote the proliferation of CD4+ and CD8+ T cells, enhance the cytotoxicity of CD8+ T cells and NK cells, and will not cause cell apoptosis due to activation. IL-21 will preferentially expand "young" CD8+ T cells with CD27+ and CD28+ , which have stronger cytotoxicity. Of course, IL-21 will not cause the expansion of Treg, so IL-21 is increasingly used in cell immunotherapy.

Specifically, interleukin 4 (IL-4): IL-4 activates the proliferation of activated B cells and T cells, and regulates the expression of Fc receptors on lymphocytes and monocytes. IL-4 induces the transformation of Th1 cells to Th2 cells. IL-4 stimulates Th2 cells to secrete IL-4, IL-5, IL-6, IL-10 and IL-13. IL-4 guides monocytes to differentiate into DCs by inhibiting the growth of macrophages. When IL-4 is not added to a culture system, monocytes will differentiate into macrophages. IL-4 plays a key role in regulating humoral immunity and adaptive immunity, inducing B cell antibody class switching to IgE and upregulating the production of MHC class II molecules. The combined action of IL-4 and GM-CSF can direct the differentiation of monocytes into immature DCs. At this time, DCs have stronger antigen uptake and processing capabilities, but weaker antigen presentation capabilities. The sequential use of IL-4 and TNF-α can promote DC maturation.

Specifically, interleukin 12 (IL-12): IL-12 acts on activated T and NK cells, has a wide range of biological activities, and acts on lymphocytes through the mediation of the activator of the transcription protein STAT4. IL-12 is required for the T cell-independent induction of IFN-γ and plays an important role in the differentiation of Th1 and Th2 cells. IL12B combines with IL23A to form IL-23 interleukin, which has innate and adaptive immune functions. IL-12 is a drug target. In cellular immunotherapy, IL-12 promotes the differentiation of CD4+ T cells into CD4+ Th1 T cells and enhances the activity of CD8+ CTLs cells. The therapeutic effect of IL-12 is related to its dose, duration of action, and other interacting cytokines, and promotes the tumor-killing activity of immune cells through multiple mechanisms. In the mouse anti-melanoma model, high-dose IL-12 acts through NK cells, while low-dose IL-12 has a tumor-killing effect through NKT.

Specifically, interleukin 18 (IL-18): IL-18 is also called interferon-γ inducing factor, which is a proinflammatory cytokine produced by macrophages and other cells. IL-18 can stimulate NK cells and CD8+ T cells to secrete IFN-γ, and enhance the cytotoxic effect of NK cells and CD8+ T cells. IL-18 can also activate macrophages, promote the development of Th1 CD4+ T cells and promote the expression of FasL by lymphocytes. IL-18 can provide a potential therapeutic target for allergic diseases. In addition, the synergistic effect of IL-18, IL-12 and IL-15 can maintain Th1 response and monokine production in autoimmune diseases.

Interferon-γ (IFN-γ) belongs to type II interferon, which is mainly produced by NK and NKT cells, has antiviral, antitumor and immunomodulatory effects and includes IFN-γ and IFN-β. IFN-γ has an anti-proliferative effect on transformed cells and can enhance the antiviral and anti-tumor effects of type I interferon. By activating macrophages, IFN-γ induces the expression of MHCI, MHCI II and co-activator molecules on antigen presenting cells (APCs). In addition, IFN-γ can induce changes in the expression of proteasomes to enhance antigen presentation ability. IFN-γ can also promote the differentiation of CD4+ T cells into Th1 cells and inhibit the subtype switching of B cells that depends on IL-4. IFN-γ activates the JAK-STAT cell pathway by phosphorylating JAK1 and JAK2 proteins. In cellular immunotherapy, IFN-γ acts on host immune cells and has certain effects on macrophages, T cells, B cells and NK cells. IFN-γ enhances antigen presentation ability by promoting the expression of MHC class II molecules in macrophages or by causing some cells that normally do not express MHC class II molecules (such as vascular endothelial cells, some epithelial cells and connective tissue cells) to express MHC class II molecules. IFN-γ can promote the differentiation of B cells and CD8+ T cells, but cannot promote their proliferation. IFN-γ can enhance the activity and immune function of TH1 cells. IFN-γ enhances the phagocytic ability of neutrophils and activates NK cells to enhance their cytotoxic effect. Abnormal expression of IFN-γ is associated with many autoinflammatory and autoimmune diseases.

Tumor necrosis factor belongs to the TNF superfamily cytokines and is a multifunctional molecule that regulates biological processes, including cell proliferation, differentiation, apoptosis, lipid metabolism and coagulation. TNF-α participates in anti-tumor. In terms of cellular immunotherapy, TNF-α causes immature DC to differentiate into mature DC. This process is achieved by TNF-α downregulating the macropinocytosis of immature DC and the expression of surface Fc receptors, and upregulating the expression of MHCI class, class II molecules and B7 family molecules (CD80, CD86, etc.) on cell surface . The antigen uptake and processing capabilities of mature DCs are significantly weakened, but their antigen presentation capabilities are significantly enhanced, which can strongly activate T cells. TNF-α can also affect the production of other cytokines, such as stimulating the secretion of IL-1 by monocytes and macrophages, enhancing the proliferation of IL-2-dependent thymocytes and T cells, promoting the production of lymphokines such as IL-2, CSF and IFN-γ, and enhancing the proliferation and Ig secretion of B cells stimulated by mitogens or foreign antigens.

Granulocyte macrophage colony-stimulating factor (GM-CSF) plays a key role in embryo transfer and development. GM-CSF is one of the earliest cytokines discovered to have an effect on DCs. In DC culture, GM-CSF promotes the differentiation of monocytes into large macrophage-like cells and promotes the expression of MHC class II molecules on the cell surface, thereby enhancing the antigen presentation function of cells. In addition, GM-CSF can also promote DC survival. In terms of cellular immunotherapy, GM-CSF can activate immune responses and produce anti-tumor activity by activating macrophages and DCs. In terms of antigen presentation, GM-CSF can promote DC cell maturation, promote the upregulation of costimulatory molecules and the expression of CD1d receptors. Recent studies have found that GM-CSF stimulates the differentiation of hematopoietic progenitor cells into monocytes and neutrophils, thereby reducing the risk of febrile neutropenia in cancer patients. Other studies have shown that GM-CSF can induce the differentiation of bone marrow DCs, promote Th1 cell-biased immune responses and angiogenesis, and affect the development of allergic inflammation and autoimmune diseases. Therefore, GM-CSF is used clinically to treat malignant tumors.

The term "nucleic acid molecule", as used herein, generally refers to nucleotides of any length. In the present application, the term "nucleic acid molecule" may encode a protein included by the oncolytic virus. In the present application, the nucleic acid molecule may include DNA and/or RNA. In some cases, the RNA may include single-stranded RNA (ssRNA) or double-stranded RNA (dsRNA). The single-stranded RNA may include sense RNA or anti-sense RNA, or it may include ambisense RNA.

In the present application, the term "expression vector" generally refers to a nucleic acid vector. Under appropriate conditions, it is usually capable of expressing a target gene and/or target protein. In certain embodiments of the present application, the expression vector includes a nucleic acid molecule for expressing one or more components of a virus (e.g., an oncolytic virus). For example, the expression vector may include at least one viral genomic element and may be packaged into a virus or into virions.

The term "virus-producing cell", as used herein, generally refers to cells, cell lines or cell cultures that can or have contained nucleic acid molecules or expression vectors described herein, or are capable of expressing recombinant oncolytic viruses described herein. The cell may include progeny of a single host cell. The cells may be obtained by in vitro transfection using the expression vector described herein.

The term "pharmaceutical composition", as used herein, generally refers to a formulation that is in a form that allows the biological activity of the active ingredient to be effective, and that contains no additional ingredients that are unacceptably toxic to a subject to which the formulation is to be administered. In certain embodiments, these formulations may include active ingredients of a medicine and a pharmaceutically acceptable carrier. In certain embodiments, the medicine product encompasses medicine products that are administered parenterally, transdermally, intracavitary, intraarterially, intrathecally, and/or intranasally, or injected directly into a tissue. The medicine product may be administered by different routes, such as an intravenous, intraperitoneal, subcutaneous, intramuscular, topical or intradermal route.

The term "prevention/preventing ", as used herein, generally refers to preventing the occurrence and onset, recurrence, and/or spread of a disease or one or more symptoms of the disease by taking certain measures in advance. The term "treatment", as used herein, generally refers to eliminating or ameliorating a disease, or one or more symptoms associated with the disease. In certain embodiments, treatment generally refers to administering one or more drugs to a patient suffering from the disease so that the disease is eliminated or alleviated. In certain embodiments, "treatment" may be the administration of the pharmaceutical composition and/or drug product in the presence or absence of other drugs after the onset of symptoms of a particular disease. For example, it may be the use of the pharmaceutical composition and/or drug product described herein to prevent the occurrence, development, recurrence and/or metastasis of a tumor.

The term "tumor", as used herein, generally refers to any new pathological growth of tissue. The tumor may be benign or malignant. In the present application, the tumor may be a solid tumor or a hematological tumor. For research use, these tissues can be isolated from readily available sources by methods well known to those skilled in the art.

### DETAILED DESCRIPTION

A wild-type VSV, in particular, an Indiana strain of the VSV, Indiana MuddSummer subtype strain of the VSV. The Indiana MuddSummer subtype strain of the VSV includes: an M protein with an amino acid sequence as shown in SEQ ID NO 1; a G protein with an amino acid sequence as shown in SEQ ID NO 12; an N protein with an amino acid sequence as shown in SEQ ID NO 14; a P protein with an amino acid sequence as shown in SEQ ID NO 16; an L protein with an amino acid sequence as shown in SEQ ID NO 18. In the present application, the M protein, G protein, N protein, P protein, and L protein all can be modified.

An oncolytic virus, based on the wild-type VSV as described above, being obtained by mutating the sites on the amino acid sequences of the M protein, G protein, N protein, P protein, and L protein.

The present application provides a recombinant oncolytic virus. The recombinant oncolytic virus is obtained by introducing an exogenous gene encoding a cytokine into the oncolytic virus as described above.

The recombinant oncolytic virus includes an M protein and a cytokine encoded by an exogenous gene, wherein the M protein includes the following site mutations compared to an amino acid sequence as shown in SEQ ID NO 1: mutating of methionine at position 51 into arginine (M51R); mutating of valine at position 221 into phenylalanine (V221F); and mutating of serine at position 226 into arginine (S226R).

The M protein further includes one or more of the following site mutations: mutating of asparagine at position 32 into serine (N32S); and/or mutating of asparagine at position 49 into aspartic acid (N49D); and/or mutating of histidine at position 54 into tyrosine (H54Y); and/or mutating of valine at position 225 into isoleucine (V225I).

The M protein further includes one or more of the following site mutations: knocking out of leucine at position 111; or, mutating of leucine at position 111 into alanine (L111A).

The M protein further includes one or more of the following site mutations: mutating of glycine at position 21 into alanine (G21E); and/or mutating of methionine at position 33 into alanine (M33A); and/or mutating of alanine at position 133 into threonine (A133T). For example, the M protein includes amino acid sequences as shown in SEQ ID NOs 2-11.

In the present application, the M protein includes amino acid substitutions at positions 51, 221, and 226.

In the present application, the M protein includes amino acid substitutions at positions 32, 49, 51, 54, 221, 225, and 226.

In the present application, the M protein includes amino acid substitutions at positions 32, 49, 51, 54, 111, 221, 225, and 226.

In the present application, the M protein includes amino acid substitutions at positions 21, 32, 49, 51, 54, 221, 225, and 226.

In the present application, the M protein includes amino acid substitutions at positions 21, 32, 33, 49, 51, 54, 221, 225, and 226.

In the present application, the M protein includes amino acid substitutions at positions 21, 32, 33, 49, 51, 54, 133, 221, 225, and 226.

In the present application, the M protein includes amino acid substitutions at positions 32, 33, 49, 51, 54, 221, 225, and 226.

In the present application, the M protein includes amino acid substitutions at positions 32, 33, 49, 51, 54, 133, 221, 225, and 226.

In the present application, the M protein includes amino acid substitutions at positions 32, 49, 51, 54, 133, 221, 225, and 226.

In the present application, the M protein may further include amino acid substitutions at other positions.

Further, the cytokine is at least one selected from a group consisting of: interleukins (IL), interferons (IFN), tumor necrosis factors (TNF), colony stimulating factors (CSF), transforming growth factor-β family (TGF-β family), chemokine family, and growth factors.

Further, the cytokine is at least one selected from a group consisting of: GM-CSF, G-CSF, M-CSF, IL-1, IL-2, IL-4, IL-5, IL-6, IL-9, IL-10, IL-12, IL-13, IL-15, IL-17, IL-18, IL-23, IL-27, IFN-α, IFN-β, IFN-γ, IFN-β, TGF-β and TNF-α.

Further, the cytokine is at least one selected from a group consisting of: GM-CSF, IL-2, IL-12, IL-15, IL-18, TNF-α and IFN-β.

A recombinant oncolytic virus, including the M protein as described above; the recombinant oncolytic virus further includes a G protein, wherein the G protein includes one or more of the following site mutations compared to an amino acid sequence as shown in SEQ ID NO 12: mutating of valine at position 53 into isoleucine (V531); and/or, mutating of alanine at position 141 into valine (A141V); and/or, mutating of aspartic acid at position 172 into tyrosine (D172Y); and/or, mutating of lysine at position 217 into glutamic acid (K217E); and/or, mutating of aspartic acid at position 232 into glycine and/or, mutating of valine at position 331 to alanine (V331A); and/or, mutating of valine at position 371 into glutamic acid (V371E); and/or, mutating of glycine at position 436 into aspartic acid (G436D); and/or, mutating of threonine at position 438 into serine (T438S); and/or, mutating of phenylalanine at position 453 into leucine (F453L); and/or, mutating of threonine at position 471 into isoleucine (T471I); and/or, mutating of tyrosine at position 487 into histidine (Y487H). For example, the G protein includes an amino acid sequence as shown in SEQ ID NO 13.

In the present application, the G protein may include amino acid mutations at positions 53, 141, 172, 217, 232, 331, 371, 436, 438, 453, 471 and 487.

In the present application, the G protein may further include amino acid substitutions at other positions.

In some embodiments, the G protein includes at least one or more amino acid substitutions in a conserved region. For example, the conserved region may includes amino acids at positions 437-461 of the G protein. In some embodiments, the G protein includes at least one or more amino acid substitutions in a truncated region of a cytoplasmic domain. For example, the truncated region of the cytoplasmic domain may include amino acids at positions 483-511 of the G protein.

A recombinant oncolytic virus, including the M protein as described above or the M protein and G protein as described above; the recombinant oncolytic virus further includes an N protein, wherein the N protein includes one or more of the following site mutations compared to an amino acid sequence as shown in SEQ ID NO 14: mutating of isoleucine at position 14 into valine (I14V); and/or, mutating of arginine at position 155 into lysine (R155K); and/or, mutating of serine at position 353 into asparagine (S353N). For example, the N protein includes an amino acid sequence as shown in SEQ ID NO 15.

In the present application, the N protein may include amino acid mutations at positions 14, 155, and 353.

In the present application, the N protein may further include amino acid substitutions at other positions.

A recombinant oncolytic virus, including the M protein as described above; or the M protein and G protein as described above; or the M protein, G protein and N protein as described above; the recombinant oncolytic virus further includes a P protein, wherein the P protein includes one or more of the following site mutations compared to an amino acid sequence as shown in SEQ ID NO 16: mutating of arginine at position 50 into lysine (R50K); and/or mutating of valine at position 76 into alanine (V76A); and/or mutating of asparagine at position 99 into glutamic acid (D99E); and/or mutating of leucine at position 126 into serine (L126S); and/or mutating of leucine at position 140 into serine (L140S); and/or mutating of histidine at position 151 into tyrosine (H151Y); and/or mutating of isoleucine at position 168 into methionine (T168M); and/or mutating of lysine at position 170 into glutamic acid (K170E); and/or mutating of tyrosine at position 189 into serine (Y189S); and/or mutating of asparagine at position 237 into aspartic acid (N237D). For example, the P protein includes an amino acid sequence as shown in SEQ ID NO 17.

In the present application, the G protein may include amino acid mutations at positions 50, 76, 99, 126, 140, 151, 168, 170, 189 and 237.

In the present application, the P protein may further include amino acid substitutions at other positions.

A recombinant oncolytic virus, including the M protein as described above; or the M protein and G protein as described above; or the M protein, G protein and N protein as described above; or the M protein, G protein, N protein and P protein as described above; the recombinant oncolytic virus further includes an L protein, wherein the L protein includes one or more of the following site mutations compared to an amino acid sequence as shown in SEQ ID NO 18: mutating of serine at position 87 into proline (S87P); and/or, mutating of isoleucine at position 487 into threonine (I487T). For example, the L protein includes an amino acid sequence as shown in SEQ ID NO 19.

In the present application, the L protein may include amino acid mutations at positions 87 and 487.

In the present application, the L protein may further include amino acid substitutions at other positions.

The recombinant oncolytic virus includes a nucleic acid molecule; the nucleic acid molecule includes a nucleic acid sequence encoding the M protein with the site mutation(s), and/or a nucleic acid sequence encoding the G protein with the site mutation(s), and/or a nucleic acid sequence encoding the N protein with the site mutation(s), and/or a nucleic acid sequence encoding the P protein with the site mutation(s), and/or a nucleic acid sequence encoding the L protein with the site mutation(s), and a nucleic acid sequence encoding the cytokine.

Further, in the nucleic acid molecule, the nucleic acid sequence encoding the cytokine is located between the nucleic acid sequence encoding the G protein with the site mutation(s) and the nucleic acid sequence encoding the L protein with the site mutation(s).

Further, in the nucleic acid molecule, the nucleic acid sequence encoding the cytokine is located between the nucleic acid sequence encoding the M protein with the site mutation(s), and/or the nucleic acid sequence encoding the N protein with the site mutation(s), and/or the nucleic acid sequence encoding the P protein with the site mutation(s), and/or the nucleic acid sequence encoding the L protein with the site mutation(s).

In the present application, the recombinant oncolytic virus described herein can be obtained through a virus packaging process and a virus rescue process. The specific process may include: infecting and inoculating BSR-T7 cells with poxvirus vTF7-3 expressing T7 RNA polymerase, and transfecting with lipofectamine using expression plasmids and backbone plasmids that clone VSV N, VSV P, and VSV L genes, respectively, to obtain a target oncolytic virus.

The present application further provides an expression vector of the recombinant oncolytic virus, a virus-producing cell, a vaccine, and a pharmaceutical composition.

The expression vector of the recombinant oncolytic virus may include nucleic acid sequences encoding the M protein and G protein of the recombinant oncolytic virus; the expression vector of the recombinant oncolytic virus may further include nucleic acid sequences encoding the N protein, P protein, and L protein of the recombinant oncolytic virus.

The virus-producing cell is capable of producing the recombinant oncolytic virus as described above; the virus-producing cell may include BSR-T7 cells, Vero cells, 293 cells, MRC-5 cells, and WI38 cells.

The vaccine is prepared by using the recombinant oncolytic virus as described above.

The pharmaceutical composition includes the recombinant oncolytic virus as described above, and optionally a pharmaceutically acceptable carrier.

In certain embodiments, the pharmaceutical composition may include a suitable formulation of one or more (pharmaceutically effective) adjuvants, stabilizers, excipients, diluents, solubilizers, surfactants, emulsifiers and/or preservatives. The acceptable ingredients of the pharmaceutical composition are preferably non-toxic to a recipient at the dose and concentration used. The pharmaceutical compositions of the present application include but are not limited to liquid, frozen and lyophilized compositions.

In certain embodiments, the pharmaceutically acceptable carrier may include any and all solvents, dispersion media, coatings, isotonic agents and absorption delaying agents that are compatible with drug administration, and are generally safe and non-toxic.

The pharmaceutical composition includes the recombinant oncolytic virus described above and, optionally, other pharmaceutically acceptable drugs. The pharmaceutical composition described above can be used for combined treatment of a disease, including but not limited to the treatment of a tumor.

In certain embodiments, the pharmaceutical composition may be useful for administration including parenteral, subcutaneous, intracavitary, intravenous, intrathecal, and/or intranasal administration or may be directly injected into tissues. For example, the pharmaceutical composition may be administered to a patient or subject by infusion or injection. In certain embodiments, the administration of the pharmaceutical composition may be performed in different ways, such as intravenous, intraperitoneal, subcutaneous, intramuscular, intradermal or intratissue administration. In certain embodiments, the pharmaceutical composition may be administered uninterruptedly. The uninterrupted (or continuous) administration may be achieved by a small pump system worn by a patient to measure a therapeutic agent flowing into the patient's body, as described in WO2015/036583.

In addition, the present application provides a method for preparing the recombinant oncolytic virus, the expression vector of the recombinant oncolytic virus, the virus-producing cell, the vaccine or the pharmaceutical composition as described above. Any method suitable for producing oncolytic viruses may be used to produce the recombinant oncolytic viruses of the present application. For example, a poxvirus expressing T7 RNA polymerase can be added to a cell for transfection, a plasmid and a backbone plasmid expressing the N protein, L protein, and P protein of the recombinant oncolytic virus can be added for transfection, and the recombinant oncolytic virus of the present application can be obtained through a virus rescue process.

The present application provides a use of the recombinant oncolytic virus, the expression vector of the recombinant oncolytic virus, the virus-producing cell, the vaccine or the pharmaceutical composition in preparing a medicine for preventing and/or treating a disease and/or disorder.

The recombinant oncolytic viruses of the present application are obtained by performing site-directed mutagenesis on the amino acids on the M protein, G protein, N protein, P protein and L protein of an oncolytic virus, respectively, thereby further improving the infective ability of the oncolytic viruses to the abnormally proliferative (tumor) LLC cell, 4T1 cell, MC38 cell and Hela cell. Moreover, the recombinant oncolytic viruses prepared have poor infective ability to normal cells (normal MEF cells), indicating that the recombinant oncolytic viruses prepared herein can be well used for infecting tumors, cancers and other cells without damaging normal cells, and have broad application prospects.

The recombinant oncolytic viruses of the present application are obtained by performing site-directed mutagenesis on the amino acids on the M protein, G protein, N protein, P protein and L protein of an oncolytic virus, respectively, thereby further improving the infective ability of the oncolytic viruses to the abnormally proliferative (tumor) LLC cell, 4T1 cell, MC38 cell and Hela cell and further improving the killing ability of the oncolytic viruses to the LLC cell, the 4T1 cell, the MC38 cell and the Hela cell in vitro. Moreover, the recombinant oncolytic viruses prepared almost have no effect on normal MEF cells, indicating that the recombinant oncolytic viruses prepared herein can be well used for damaging and killing abnormal cells such as tumor and cancer cells, without causing damage normal cells.

The recombinant oncolytic viruses of the present application are not easy to eliminate in the abnormally proliferative (tumor) LLC cell, 4T1 cell, MC38 cell and Hela cell. In contrast, wild-type oncolytic viruses are easier to eliminate in the LLC cell, the MC38 cell and the Hela cell. The recombinant oncolytic viruses of the present application are obtained by performing site-directed mutagenesis on the amino acids on the M protein, G protein, N protein, P protein, and L protein of an oncolytic virus, respectively, so that the oncolytic viruses can be more difficult to eliminate in the LLC cell, the 4T1 cell, the MC38 cell, and the Hela cell, thereby further ensuring that the oncolytic viruses can better exert the infective ability and killing ability to the LLC cell, the 4T1 cell, the MC38 cell, and the Hela cell. Moreover, the recombinant oncolytic viruses of the present application are easier to eliminate in normal MEF cells and the safety of the normal MEF cells is further ensured, thereby improving the safety of the recombinant oncolytic viruses.

The present disclosure is further described in detail below in combination with Preparation Examples 1-106 and Examples 1-3.

### PREPARATION EXAMPLES

### Preparation Examples 1-70

Preparation Examples 1-70 respectively provide a recombinant oncolytic virus. The recombinant oncolytic virus includes an M protein and a cytokine. Specifically as follows.

The cytokine included in the recombinant oncolytic virus provided in each of Preparation Examples 1-10 is GM-CSF which includes an amino acid sequence as shown in SEQ ID NO 20. And these preparation examples differ in the positions of amino acids having site-directed mutagenesis on the M protein of a wild-type oncolytic virus, as shown in Table 1.

The cytokine included in in the recombinant oncolytic virus provided in each of Preparation Examples 11-20 is IL-2 which includes an amino acid sequence as shown in SEQ ID NO 21. And these preparation examples differ in the positions of amino acids having site-directed mutagenesis on the M protein of a wild-type oncolytic virus, as shown in Table 1.

The cytokine included in the recombinant oncolytic virus provided in each of Preparation Examples 21-30 is IL-12 which includes amino acid sequences as shown in SEQ ID NOs 22 and 23. And these preparation examples differ in the positions of amino acids having site-directed mutagenesis on the M protein of a wild-type oncolytic virus, as shown in Table 1.

The cytokine included in the recombinant oncolytic virus provided in each of Preparation Examples 31-40 is IL-15 which includes an amino acid sequence as shown in SEQ ID NO 24. And these preparation examples differ in the positions of amino acids having site-directed mutagenesis on the M protein of a wild-type oncolytic virus, as shown in Table 1.

The cytokine included in the recombinant oncolytic virus provided in each of Preparation Examples 41-50 is IL-18 which includes an amino acid sequence as shown in SEQ ID NO 25. And these preparation examples differ in the positions of amino acids having site-directed mutagenesis on the M protein of a wild-type oncolytic virus, as shown in Table 1.

The cytokine included in the recombinant oncolytic virus provided in each of Preparation Examples 51-60 is TNF-α which includes an amino acid sequence as shown in SEQ ID NO 26. And these preparation examples differ in the positions of amino acids having site-directed mutagenesis on the M protein of a wild-type oncolytic virus, as shown in Table 1.

The cytokine included in the recombinant oncolytic virus provided in each of Preparation Examples 61-70 is IFN-β which includes an amino acid sequence as shown in SEQ ID NO 27. And these preparation examples differ in the positions of amino acids having site-directed mutagenesis on the M protein of a wild-type oncolytic virus, as shown in Table 1.

A construction method for the recombinant oncolytic virus provided by each of the above preparation examples is as follows.

### (1) Construction of vector

Using a pRV-core plasmid (BioVector NTCC Plasmid Vector Cell Gene Collection Center) as a template, the mutation sites of the M protein as shown in Table 1 were introduced by PCR technology.

A gene fragment containing XbaI and MluI restriction sites and including the protein mutation sites was synthesized. PCR amplification was performed using the gene fragment as a template. The PCR product was subjected to 1% agarose gel electrophoresis and double-digested with XbaI and MluI, and a gene fragment with the protein mutation sites was obtained by gel extraction using a gel extraction kit.

The pRV-core plasmid was double-digested with XbaI and Mlu I, and a pRV-core restriction digestion extraction backbone fragment was obtained by gel extraction using a gel extraction kit.

The gene fragment with the protein mutation sites and the pRV-core restriction digestion extraction backbone fragment were connected and transformed and then plated, and monoclones were selected and shaken for identification by PCR. The plasmid was extracted to obtain the constructed plasmid pRV-core Mut, which was sent to a sequencing company for sequencing.

### (2) Insertion of exogenous gene

The plasmid pRV-core Mut obtained in step (1) was double-digested with Xho I and Mlu I to extract a long fragment.

An exogenous gene encoding the cytokine was synthesized by a gene synthesis company and amplified with corresponding primers. The amplification product was double-digested with XhoI and NheI and a target gene fragment was extracted. The double-digested pRV-core Mut and the exogenous gene fragment were connected and transformed. Monoclones were selected and identified by PCR or enzyme digestion, and then sequenced by a sequencing company, as shown in Table 1, to obtain a plasmid pRV-core Mut carrying the exogenous gene.

**Table 1 Mutations of the recombinant oncolytic viruses in Preparation Examples 1-70**

| Preparation Example | M protein | | | Cytokine | |
|---|---|---|---|---|---|
| | Mutation site and amino acid after mutation | Number of mutations | Sequence | Type | Sequence |
| 1 | M51R, V221F, S226R | 3 | SEQ ID NO 2 | GM-CSF | SEQ ID NO 20 |
| 2 | N32S, N49D, M51R, H54Y, V221F, V225I, S226R | 7 | SEQ ID NO 3 | | |
| 3 | N32S, N49D, M51R, H54Y, knocking out of leucine at position 111, V221F, V225I, S226R | 8 | SEQ ID NO 4 | | |
| 4 | N32S, N49D, M51R, H54Y, L111A, V221F, V225I, S226R | 8 | SEQ ID NO 5 | | |
| 5 | G21E, N32S, N49D, M51R, H54Y, V221F, V225I, S226R | 8 | SEQ ID NO 6 | | |
| 6 | G21E, N32S, M33A, N49D, M51R, H54Y, V221F, V225I, S226R | 9 | SEQ ID NO 7 | | |
| 7 | G21E, N32S, M33A, N49D, M51R, H54Y, A133T, V221F, V225I, S226R | 10 | SEQ ID NO 8 | | |
| 8 | N32S, M33A, N49D, M51R, H54Y, V221F, V225I, S226R | 8 | SEQ ID NO 9 | | |
| 9 | N32S, M33A, M51R, H54Y, A133T, V221F, V225I, S226R | 9 | SEQ ID NO 10 | | |
| 10 | N32S, N49D, M51R, H54Y, A133T, V221F, V225I, S226R | 8 | SEQ ID NO 11 | | |
| 11 | M51R, V221F, S226R | 3 | SEQ ID NO 2 | IL-2 | SEQ ID NO 21 |
| 12 | N32S, N49D, M51R, H54Y, V221F, V225I, S226R | 7 | SEQ ID NO 3 | | |
| 13 | N32S, N49D, M51R, H54Y, knocking out of leucine at position 111, V221F, V225I, S226R | 8 | SEQ ID NO 4 | | |
| 14 | N32S, N49D, M51R, H54Y, L111A, V221F, V225I, S226R | 8 | SEQ ID NO 5 | | |
| 15 | G21E, N32S, N49D, M51R, H54Y, V221F, V225I, S226R | 8 | SEQ ID NO 6 | | |
| 16 | G21E, N32S, M33A, N49D, M51R, H54Y, V221F, V225I, S226R | 9 | SEQ ID NO 7 | | |
| 17 | G21E, N32S, M33A, N49D, M51R, H54Y, A133T, V221F, V225I, S226R | 10 | SEQ ID NO 8 | | |
| 18 | N32S, M33A, N49D, M51R, H54Y, V221F, V225I, S226R | 8 | SEQ ID NO 9 | | |
| 19 | N32S, M33A, N49D, M51R, H54Y, A133T, V221F, V225I, S226R | 9 | SEQ ID NO 10 | | |
| 20 | N32S, N49D, M51R, H54Y, A133T, V221F, V225I, S226R | 8 | SEQ ID NO 11 | | |
| 21 | M51R, V221F, S226R | 3 | SEQ ID NO 2 | IL-12 | SEQ ID NO 22, SEQ ID NO 23 |
| 22 | N32S, N49D, M51R, H54Y, V221F, V225I, S226R | 7 | SEQ ID NO 3 | | |
| 23 | N32S, N49D, M51R, H54Y, knocking out of leucine at position 111, V221F, V225I, S226R | 8 | SEQ ID NO 4 | | |
| 24 | N32S, N49D, M51R, H54Y, L111A, V221F, V225I, S226R | 8 | SEQ ID NO 5 | | |
| 25 | G21E, N32S, N49D, M51R, H54Y, V221F, V225I, S226R | 8 | SEQ ID NO 6 | | |
| 26 | G21E, N32S, M33A, N49D, M51R, H54Y, V221F, V225I, S226R | 9 | SEQ ID NO 7 | | |
| 27 | G21E, N32S, M33A, N49D, M51R, H54Y, A133T, V221F, V225I, S226R | 10 | SEQ ID NO 8 | | |
| 28 | N32S, M33A, N49D, M51R, H54Y, V221F, V225I, S226R | 8 | SEQ ID NO 9 | | |
| 29 | N32S, M33A, N49D, M51R, H54Y, A133T, V221F, V225I, S226R | 9 | SEQ ID NO 10 | | |
| 30 | N32S, N49D, M51R, H54Y, A133T, V221F, V225I, S226R | 8 | SEQ ID NO 11 | | |
| 31 | M51R, V221F, S226R | 3 | SEQ ID NO 2 | | |
| 32 | N32S, N49D, M51R, H54Y, V221F, V225I, S226R | 7 | SEQ ID NO 3 | | |
| 33 | N32S, N49D, M51R, H54Y, knocking out of leucine at position 111, V221F, V225I, S226R | 8 | SEQ ID NO 4 | | |
| 34 | N32S, N49D, M51R, H54Y, L111A, V221F, V225I, S226R | 8 | SEQ ID NO 5 | | |
| 35 | G21E, N32S, N49D, M51R, H54Y, V221F, V225I, S226R | 8 | SEQ ID NO 6 | | |
| 36 | G21E, N32S, M33A, N49D, M51R, H54Y, V221F, V225I, S226R | 9 | SEQ ID NO 7 | | |
| 37 | G21E, N32S, M33A, N49D, M51R, H54Y, A133T, V221F, V225I, S226R | 10 | SEQ ID NO 8 | | |
| 38 | N32S, M33A, N49D, M51R, H54Y, V221F, V225I, S226R | 8 | SEQ ID NO 9 | | |
| 39 | N32S, M33A, N49D, M51R, H54Y, A133T, V221F, V225I, S226R | 9 | SEQ ID NO 10 | | |
| 40 | N32S, N49D, M51R, H54Y, A133T, V221F, V225I, S226R | 8 | SEQ ID NO 11 | | |
| 41 | M51R, V221F, S226R | 3 | SEQ ID NO 2 | IL-18 | SEQ ID NO 25 |
| 42 | N32S, N49D, M51R, H54Y, V221F, V225I, S226R | 7 | SEQ ID NO 3 | | |
| 43 | N32S, N49D, M51R, H54Y, knocking out of leucine at position 111, V221F, V225I, S226R | 8 | SEQ ID NO 4 | | |
| 44 | N32S, N49D, M51R, H54Y, L111A, V221F, V225I, S226R | 8 | SEQ ID NO 5 | | |
| 45 | G21E, N32S, N49D, M51R, H54Y, V221F, V225I, S226R | 8 | SEQ ID NO 6 | | |
| 46 | G21E, N32S, M33A, N49D, M51R, H54Y, V221F, V225I, S226R | 9 | SEQ ID NO 7 | | |
| 47 | G21E, N32S, M33A, N49D, M51R, H54Y, A133T, V221F, V225I, S226R | 10 | SEQ ID NO 8 | | |
| 48 | N32S, M33A, N49D, M51R, H54Y, V221F, V225I, S226R | 8 | SEQ ID NO 9 | | |
| 49 | N32S, M33A, N49D, M51R, H54Y, A133T, V221F, V225I, S226R | 9 | SEQ ID NO 10 | | |
| 50 | N32S, N49D, M51R, H54Y, A133T, V221F, V225I, S226R | 8 | SEQ ID NO 11 | | |
| 51 | M51R, V221F, S226R | 3 | SEQ ID NO 2 | TNF-α | SEQ ID NO26 |
| 52 | N32S, N49D, M51R, H54Y, V221F, V225I, S226R | 7 | SEQ ID NO 3 | | |
| 53 | N32S, N49D, M51R, H54Y, knocking out of leucine at position 111, V221F, V225I, S226R | 8 | SEQ ID NO 4 | | |
| 54 | N32S, N49D, M51R, H54Y, L111A, V221F, V225I, S226R | 8 | SEQID NO 5 | | |
| 55 | G21E, N32S, N49D, M51R, H54Y, V221F, V225I, S226R | 8 | SEQ ID NO 6 | | |
| 56 | G21E, N32S, M33A, N49D, M51R, H54Y, V221F, V225I, S226R | 9 | SEQ ID NO 7 | | |
| 57 | G21E, N32S, M33A, N49D, M51R, H54Y, A133T, V221F, V225I, S226R | 10 | SEQ ID NO 8 | | |
| 58 | N32S, M33A, N49D, M51R, H54Y, V221F, V225I, S226R | 8 | SEQID NO 9 | | |
| 59 | N32S, M33A, N49D, M51R, H54Y, A133T, V221F, V225I, S226R | 9 | SEQ ID NO 10 | | |
| 60 | N32S, N49D, M51R, H54Y, A133T, V221F, V225I, S226R | 8 | SEQ ID NO 11 | | |
| 61 | M51R, V221F, S226R | 3 | SEQ ID NO 2 | IFN-β | SEQ ID NO 27 |
| 62 | N32S, N49D, M51R, H54Y, V221F, V225I, S226R | 7 | SEQ ID NO 3 | | |
| 63 | N32S, N49D, M51R, H54Y, knocking out of leucine at position 111, V221F, V225I, S226R | 8 | SEQ ID NO 4 | | |
| 64 | N32S, N49D, M51R, H54Y, L111A, V221F, V225I, S226R | 8 | SEQ ID NO 5 | | |
| 65 | G21E, N32S, N49D, M51R, H54Y, V221F, V225I, S226R | 8 | SEQ ID NO 6 | | |
| 66 | G21E, N32S, M33A, N49D, M51R, H54Y, V221F, V225I, S226R | 9 | SEQ ID NO 7 | | |
| 67 | G21E, N32S, M33A, N49D, M51R, H54Y, A133T, V221F, V225I, S226R | 10 | SEQ ID NO 8 | | |
| 68 | N32S, M33A, N49D, M51R, H54Y, V221F, V225I, S226R | 8 | SEQ ID NO 9 | | |
| 69 | N32S, M33A, N49D, M51R, H54Y, A133T, V221F, V2251, S226R | 9 | SEQ ID NO 10 | | |
| 70 | N32S, N49D, M51R, H54Y, A133T, V221F, V225I, S226R | 8 | SEQ ID NO 11 | | |

### (2) Virus rescue

The plasmid pRV-core Mut carrying the exogenous gene was transfected into a BSR-T7 cell (purchased from ATCC, American Type Culture Collection) by cell transfection technology using a calcium phosphate transfection kit (Thermo Fisher Scientific).

Four plasmids, i.e., the pRV-core Mut, pP, pN, and pL, were mixed at a ratio of 10:5:4:1, and the total amount of the plasmids was 5 µg; the plasmid mixture was then diluted with 200 µl of an Opti-MEM (Thermo Fisher Scientific), and 7.5 µl of a transfection reagent Plus Reagent (Life Technologies) was added to obtain a transfection plasmid premix, where the pP was a plasmid carrying the phosphoprotein genes of a rhabdovirus, the pN was a plasmid carrying the nucleoprotein genes of a rhabdovirus, and the pL was a plasmid carrying the polymerase protein genes of a rhabdovirus; parent vectors corresponding to the three plasmids pN, pP, and pL were all pCAGGS (purchased from ATCC).

10 µl of Lipofectamine LTX (Thermo Fisher Scientific) was diluted with 200 µl of the Opti-MEM to obtain an LTX mixed solution.

Plasmid transfection was performed according to the method described in the instruction manual of Lipofectamine LTX. Six hours later, the BSR-T7 cell was washed twice with PBS and then inoculated in DMEM (Thermo Fisher Scientific) supplemented with 10% fetal bovine serum and incubated for three days.

The cell supernatant of the BSR-T7 cell culture solution was transferred to a Vero cell (Thermo Fisher Scientific), and the Vero cell was incubated at 37 °C for three days. The cells were observed for green fluorescence under a fluorescence microscope to determine the result of virus rescue. The rescued mutant rhabdovirus library was further passaged through the Vero cell, and monoclonal virus strains were selected in an established plaque screening system.

(3) Gene sequencing. The viral genomic RNA was extracted using a Trizol kit, and reverse transcription was performed using random primers. The reverse transcribed cDNA was subjected to PCR using primers designed for the gene sequence of the M protein and primers designed for the gene sequence encoding the cytokine.

The primer sequences designed for the gene sequence of the M protein were:
PF: ATGAGTTCCTTAAAGAA (SEQ ID NO 33);
PR: TCATTTGAAGTGG (SEQ ID NO 34).

The primer sequences designed for the gene sequence encoding the cytokines were:
GMCSF F: CCCTCGAGATGTGGCTGCAGAGCCT (SEQ ID NO 35),
GMCSF R: CGGCTAGCTCACTCCTGGACTGGCTCC (SEQ ID NO 36).
IL-2 F: CCGATGTACAGGATGCAACTCC (SEQ ID NO 37),
IL-2 R: CGGCTAGCTCAAGTCAGTGTTG (SEQ ID NO 38).
IL12 F: CCCTCGAGATGTGGCCCCCTGGGT (SEQ ID NO 39),
IL12 R: CGGCTAGCTTAACTGCAGGGCACAGATG (SEQ ID NO 40).
IL-15 F: CCGCTCGAGATGAGAATTTCGAAACC (SEQ ID NO 41),
IL-15 R: CGGCTAGCTCAAGAAGTGTTGATGAAC (SEQ ID NO 42).
IL-18 F: CCCTCGAGATGGCTGCTGAACCAGTAG (SEQ ID NO 43),
IL-18 R: CGGCTAGCCTAGTCTTCGTTTTGAAC (SEQ ID NO 44).
TNFa F: CCGCTCGAGATGAGCACTGAAAGC (SEQ ID NO 45),
TNFα R: CGGCTAGCTCACAGGGCAATGATCC (SEQ ID NO 46).
IFNβ F: CCTCGAGATGACCAACAAGTGTC (SEQ ID NO 47),
IFNβ R: CGGCTAGCTCAGTTTCGGAGG (SEQ ID NO 48).

The product was extracted after 1% agarose gel electrophoresis and sequenced by a sequencing company. The sequencing results are shown in Table 1.

### Preparation Examples 71-77

Preparation Examples 71-77 respectively provide a recombinant oncolytic virus. The recombinant oncolytic virus includes an M protein, a G protein and a cytokine. The mutation sites of the M protein are the same as the corresponding mutation sites in Preparation Example 2, and the G protein includes an amino acid sequence as shown in SEQ ID NO 13, as shown in Table 2. These preparation examples differ in the type of the cytokine contained, as shown in Table 2.

The construction method of the recombinant oncolytic viruses provided in the above preparation examples is the same as the construction method in Preparation Example 7, except that: the recombinant oncolytic viruses further include a G protein with mutation sites as shown in Table 2. The differences in the construction method are specifically as follows:
the mutation sites as shown in Table 2 were introduced by PCR technology in construction of plasmid in step (1);
gene sequencing of the G protein was performed in step (3). The viral genomic RNA was extracted using a Trizol kit, and reverse transcription was performed using random primers. The reverse transcribed cDNA was subjected to PCR using primers designed for the gene sequence of the G protein;
the primer sequences designed for the gene sequence of the G protein were:
   PF: ATGAAGTGCCTTTTGTACTTAG (SEQ ID NO 49);
   PR: TTACTTTCCAAGTCGGTTCATCT (SEQ ID NO 50).

The product was extracted after 1% agarose gel electrophoresis and sequenced by a sequencing company. The sequencing results are shown in Table 2.

**Table 2 Mutations of the recombinant oncolytic viruses in Preparation Examples 71-77**

| Preparation | | G protein | | | Cytokine |
|---|---|---|---|---|---|
| Example | Mutation site and amino acid after mutation | Number of mutations | Sequence | Type | Sequence |
| 71 | | | | GM-CSF | SEQ ID NO 20 |
| 72 | V53I, A141V, D172Y, K217E, D232G, V331A, V371E, G436D, T438S, F453L, T471I, Y487H | | SEQ ID NO 13 | IL-2 | SEQ ID NO 21 |
| 73 | | 12 | | IL-12 | SEQ ID NO 22, SEQ ID NO 23 |
| 74 | | | | IL-15 | SEQ ID NO 24 |
| 75 | | | | IL-18 | SEQ ID NO 25 |
| 76 | | | | TNF-α | SEQ ID NO 26 |
| 77 | | | | IFN-β | SEQ ID NO 27 |

### Preparation Examples 78-84

Preparation Examples 78-84 respectively provide a recombinant oncolytic virus. The recombinant oncolytic virus includes an M protein, a G protein, an N protein and a cytokine. The mutation sites of the M protein and the G protein are the same as the corresponding mutation sites in Preparation Example 71, and the N protein includes an amino acid sequence as shown in SEQ ID NO 15, as shown in Table 3. These preparation examples differ in the type of the cytokine contained, as shown in Table 3.

The construction method of the recombinant oncolytic viruses provided in the above preparation examples is the same as the construction method in Preparation Example 71, except that: the recombinant oncolytic viruses further include an N protein with mutation sites as shown in Table 3. The differences in the construction method are specifically as follows:
the mutation sites as shown in Table 3 were introduced by PCR technology in construction of plasmid in step (1);
gene sequencing of the N protein was performed in step (3). The viral genomic RNA was extracted using a Trizol kit, and reverse transcription was performed using random primers. The reverse transcribed cDNA was subjected to PCR using primers designed for the gene sequence of the N protein;
the primer sequences designed for the gene sequence of the N protein were:
   PF: ATGTCTGTTACAGTCAAGAG (SEQ ID NO 51);
   PR: TCATTTGTCAAATTCTGACTT (SEQ ID NO 52).

The product was extracted after 1% agarose gel electrophoresis and sequenced by a sequencing company. The sequencing results are shown in Table 3.

**Table 3 Mutations of the recombinant oncolytic viruses in Preparation Examples 78-84**

| Preparation Example | N protein | | | Cytokine | |
|---|---|---|---|---|---|
| | Mutation site and amino acid after mutation | Number of mutations | Sequence | Type | Sequence |
| 78 | I14V, R155K, S353N | 3 | SEQ ID NO 15 | GM-CSF | SEQ ID NO 20 |
| 79 | | | | IL-2 | SEQ ID NO 21 |
| 80 | | | | IL-12 | SEQ ID NO 22, SEQ ID NO 23 |
| 81 | | | | IL-15 | SEQ ID NO 24 |
| 82 | | | | IL-18 | SEQ ID NO 25 |
| 83 | | | | TNF-α | SEQ ID NO 26 |
| 84 | | | | IFN-β | SEQ ID NO 27 |

### Preparation Examples 85-91

Preparation Examples 85-91 respectively provide a recombinant oncolytic virus. The recombinant oncolytic virus includes an M protein, a G protein, an N protein, a P protein and a cytokine. The mutation sites of the M protein, the G protein and the P protein are the same as the corresponding mutation sites in Preparation Example 78, and the P protein includes an amino acid sequence as shown in SEQ ID NO 17, as shown in Table 4. These preparation examples differ in the type of the cytokine contained, as shown in Table 4.

The construction method of the recombinant oncolytic viruses provided in the above preparation examples is the same as the construction method in Preparation Example 78, except that: the recombinant oncolytic viruses further include a P protein with mutation sites as shown in Table 4. The differences in the construction method are specifically as follows:
the mutation sites as shown in Table 4 were introduced by PCR technology in construction of plasmid in step (1);
gene sequencing of the P protein was performed in step (3). The viral genomic RNA was extracted using a Trizol kit, and reverse transcription was performed using random primers. The reverse transcribed cDNA was subjected to PCR using primers designed for the gene sequence of the P protein;
the primer sequences designed for the gene sequence of the P protein were:
   PF: ATGGATAATCTCACAAAAGTTCG (SEQ ID NO 53);
   PR: CTACAGAGAATATTTGACTCTCG (SEQ ID NO 54).

The product was extracted after 1% agarose gel electrophoresis and sequenced by a sequencing company. The sequencing results are shown in Table 4.

**Table 4 Mutations of the recombinant oncolytic viruses in Preparation Examples 85-91**

| Preparation Example | P protein | | | Cytokine | |
|---|---|---|---|---|---|
| | Mutation site and amino acid after mutation | Number of mutations | Sequence | Type | Sequence |
| 85 | R50K, V76A, D99E, L126S, L140S, H151Y, I168M, K170E, Y189S, N237D | 10 | SEQ ID NO 17 | GM-CSF | SEQ ID NO 20 |
| 86 | | | | IL-2 | SEQ ID NO 21 |
| 87 | | | | IL-12 | SEQ ID NO 22, SEQ ID NO 23 |
| 88 | | | | IL-15 | SEQ ID NO 24 |
| 89 | | | | IL-18 | SEQ ID NO 25 |
| 90 | | | | TNF-α | SEQ ID NO 26 |
| 91 | | | | IFN-β | SEQ ID NO 27 |

### Preparation Examples 92-98

Preparation Examples 92-98 respectively provide a recombinant oncolytic virus. The recombinant oncolytic virus includes an M protein, a G protein, an N protein, a P protein, an L protein and a cytokine. The mutation sites of the M protein, the G protein, the P protein and the N protein are the same as the corresponding mutation sites in Preparation Example 85, and the L protein includes an amino acid sequence as shown in SEQ ID NO 19, as shown in Table 5. These preparation examples differ in the type of the cytokine contained, as shown in Table 5.

The construction method of the recombinant oncolytic viruses provided in the above preparation examples is the same as the construction method in Preparation Example 85, except that: the recombinant oncolytic viruses further include an L protein with mutation sites as shown in Table 5. The differences in the construction method are specifically as follows:
the mutation sites as shown in Table 5 were introduced by PCR technology in construction of plasmid in step (1);
gene sequencing of the L protein was performed in step (3). The viral genomic RNA was extracted using a Trizol kit, and reverse transcription was performed using random primers. The reverse transcribed cDNA was subjected to PCR using primers designed for the gene sequence of the L protein;
the primer sequences designed for the gene sequence of the L protein were:
   PF: ATGGAAGTCCACGATTTTGAGA (SEQ ID NO 55);
   PR:TTAATCTCTCCAAGAGTTTTCCT (SEQ ID NO 56).

The product was extracted after 1% agarose gel electrophoresis and sequenced by a sequencing company. The sequencing results are shown in Table 5.

**Table 5 Mutations of the recombinant oncolytic viruses in Preparation Examples 92-98**

| Preparation Example | L protein | | | Cytokine | |
|---|---|---|---|---|---|
| | Mutation site and amino acid after mutation | Number of mutations | Sequence | Type | Sequence |
| 92 | S87P, I487T | 2 | SEQ ID NO 19 | GM-CSF | SEQ ID NO 20 |
| 93 | | | | IL-2 | SEQ ID NO 21 |
| 94 | | | | IL-12 | SEQ ID NO 22, SEQ ID NO 23 |
| 95 | | | | IL-15 | SEQ ID NO 24 |
| 96 | | | | IL-18 | SEQ ID NO 25 |
| 97 | | | | TNF- α | SEQ ID NO 26 |
| 98 | | | | IFN-β | SEQ ID NO 27 |

### Preparation Examples 99-105

Preparation Examples 99-105 respectively provide a recombinant oncolytic virus. The recombinant oncolytic virus is obtained by introducing an exogenous gene encoding the cytokine into a wild-type oncolytic virus.

A construction method for the recombinant oncolytic virus provided by each of the above preparation examples is as follows:

### (1) Insertion of exogenous gene

Using a pRV-core plasmid (BioVector NTCC Plasmid Vector Cell Gene Collection Center) as a template, double restriction digestion with XhoI and Mlu I was performed and then an exogenous gene encoding the cytokine was inserted (the exogenous gene encoding the cytokine was synthesized by a gene synthesis company and amplified with corresponding primers). The amplification product was double-digested with XhoI and NheI and a target gene fragment was extracted. The double-digested pRV-core and the exogenous gene fragment were connected and transformed. Monoclones were selected and identified by PCR or enzyme digestion, and then sequenced by a sequencing company, as shown in Table 6, to obtain a plasmid pRV-core Mut carrying the exogenous gene.

### (2) Virus rescue

The plasmid pRV-core Mut carrying the exogenous gene was transfected into a BSR-T7 cell (purchased from ATCC, American Type Culture Collection) by cell transfection technology using a calcium phosphate transfection kit (Thermo Fisher Scientific).

Four plasmids, i.e., the pRV-core Mut, pP, pN, and pL, were mixed at a ratio of 10:5:4:1, and the total amount of the plasmids was 5 µg; the plasmid mixture was then diluted with 200 µl of an Opti-MEM (Thermo Fisher Scientific), and 7.5 µl of a transfection reagent Plus Reagent (Life Technologies) was added to obtain a transfection plasmid premix, where the pP was a plasmid carrying the phosphoprotein genes of a rhabdovirus, the pN was a plasmid carrying the nucleoprotein genes of a rhabdovirus, and the pL was a plasmid carrying the polymerase protein genes of a rhabdovirus; parent vectors corresponding to the three plasmids pN, pP, and pL were all pCAGGS (purchased from ATCC).

10 µl of Lipofectamine LTX (Thermo Fisher Scientific) was diluted with 200 µl of the Opti-MEM to obtain an LTX mixed solution.

Plasmid transfection was performed according to the method described in the instruction manual of Lipofectamine LTX. Six hours later, the BSR-T7 cell was washed twice with PBS and then inoculated in DMEM (Thermo Fisher Scientific) supplemented with 10% fetal bovine serum and incubated for three days.

The cell supernatant of the BSR-T7 cell culture solution was transferred to a Vero cell (Thermo Fisher Scientific), and the Vero cell was incubated at 37 °C for three days. The cells were observed for green fluorescence under a fluorescence microscope to determine the result of virus rescue. The rescued mutant rhabdovirus library was further passaged through the Vero cell, and monoclonal virus strains were selected in an established plaque screening system.

(3) Gene sequencing. The viral genomic RNA was extracted using a Trizol kit, and reverse transcription was performed using random primers. The reverse transcribed cDNA was subjected to PCR using primers (same as the primer sequences shown in Example 1) designed for the gene sequence encoding the cytokine.

The product was extracted after 1% agarose gel electrophoresis and sequenced by a sequencing company. The sequencing results are shown in Table 6.

**Table 6 Mutations of the recombinant oncolytic viruses in Preparation Examples 99-105**

| Preparation Example | Oncolytic virus | Cytokine | |
|---|---|---|---|
| | | Type | Sequence |
| 99 | Wild-type oncolytic virus | GM-CSF | SEQ ID NO 20 |
| 100 | | IL-2 | SEQ ID NO 21 |
| 101 | | IL-12 | SEQ ID NO 22, SEQ ID NO 23 |
| 102 | | IL-15 | SEQ ID NO 24 |
| 103 | | IL-18 | SEQ ID NO 25 |
| 104 | | TNF- α | SEQ ID NO 26 |
| 105 | | IFN-β | SEQ ID NO 27 |

### Preparation Example 106

This preparation example provides a packaging process of the recombinant oncolytic virus prepared in any one of the preparation examples 1-105 as described above, specifically including the following steps:
1) Infection and inoculation of a BSR-T7 cell (purchased from ATCC) with a poxvirus vTF7-3 (BioVector NTCC Plasmid Vector Cell Gene Collection Center) expressing T7 RNA polymerase.

Specifically, the BSR-T7 cell was plated on a 6-well plate, with 3×10⁵ cells/well; 14-16 hours after plating, the poxvirus vTF7-3 expressing the T7 RNA polymerase was added to infect the BSR-T7 cell; 6 hours after infection, the BSR-T7 cell was rinsed once with DPBS for transfection later.

### 2) Transfection process

Specifically: four plasmids, the pRV-core Mut, pP, pN, and pL, were mixed at a ratio of 10:5:4:1, and the total amount of the plasmids was 5 µg; the plasmid mixture was then diluted with 200 µl of an Opti-MEM (Thermo Fisher Scientific), and 7.5 µl of a transfection reagent Plus Reagent (Life Technologies) was added to obtain a transfection plasmid premix, where the pP was a plasmid carrying the phosphoprotein genes of a rhabdovirus, the pN was a plasmid carrying the nucleoprotein genes of a rhabdovirus, and the pL was a plasmid carrying the polymerase protein genes of a rhabdovirus; parent vectors corresponding to the three plasmids pN, pP, and pL were all pCAGGS (purchased from ATCC);
10 µl of Lipofectamine LTX (Thermo Fisher Scientific) was diluted with 200 µl of the Opti-MEM to obtain an LTX mixed solution;
200 µl of the LTX mixture was mixed with 200 µl of the transfection plasmid premix, followed by incubation at room temperature for 15 min, to obtain an LTX-DNA mixed solution;
The DPBS in the 6-well plate in step 1) was replaced with the Opti-MEM, the LTX-DNA mixed solution was dropwise added to the 6-well plate in which the BSR-T7 cell was incubated, and the 6-well plate was then shaken gently to evenly distribute the LTX-DNA mixed solution in the 6-well plate; 6-8 hours after transfection, the transfection reagent was removed and 3 ml of fresh complete medium (Thermo Fisher Scientific) was then added; 72 hours later, the cell supernatant of the BSR-T7 cell was harvested and filtered with a 0.22 µm filter to obtain the recombinant oncolytic viruses respectively corresponding to Preparation Examples 1-105.

### EXAMPLES

### Example 1

In this example, an infective ability of the recombinant oncolytic viruses prepared in Preparation Examples 1-105 and the wild-type oncolytic virus to different cells were respectively detected.

A detection used was a TCID50 detection method, that is, 200 pfu of each of the recombinant oncolytic viruses prepared in Preparation Examples 1-105 and 200 pfu of the wild-type oncolytic virus were added to culture solutions of different cells, and the 50% tissue culture infective dose (TCID50) generated of each oncolytic virus was detected.

The cells tested included: LLC cell (a mouse lung cancer cell line), 4T1 cell (a mouse breast cancer cell line), MC38 cell (a mouse colon cancer cell line), Hela cell (a human cervical cancer cell line), and MEF cell (a human fibroblast cell line).

Specifically, the detection method included the following steps.
(1) 3 mL of Vero (LLC/4T1/MC38/Hela/MEF) cell suspension was added to a 6-well plate, with 4×10⁵ cells/well, a total of 6 wells, including 2 wells for the MEF cell; the 6-well culture plate was cultured for 16 hours under an environmental condition of 37°C and 5% of CO₂;
(2) 200 pfu of each of the recombinant oncolytic viruses prepared in the preparation examples was added to each well of the 6-well culture plate; 24 hours later, 100 µl of the supernatant of the MEF cell and 100 µl of the supernatant of each Vero cell were harvested; the harvested supernatants were added to the wells of a 96-well plate, with a density of each type of cell being 1×10⁴ cells/ml; and the 96-well plate was then cultured for 16 hours in the environmental condition of 37°C and 5% of CO₂s;
(3) the supernatants harvested in step (2) was diluted at a 10-fold dilution ratio serially in a 1.5m LEP tube, a total of 11 titers ranging from 10⁻¹ to 10⁻¹¹ were obtained; the diluted supernatants were inoculated into 96-well culture plates, a column of 8 wells for each titer, 100 µl/well;
(4) 48 hours later, the cells in each well were observed for fluorescence, and the wells in which the cells were observed fluorescent were recorded as infected. TCID50 was calculated according to a Karber method.

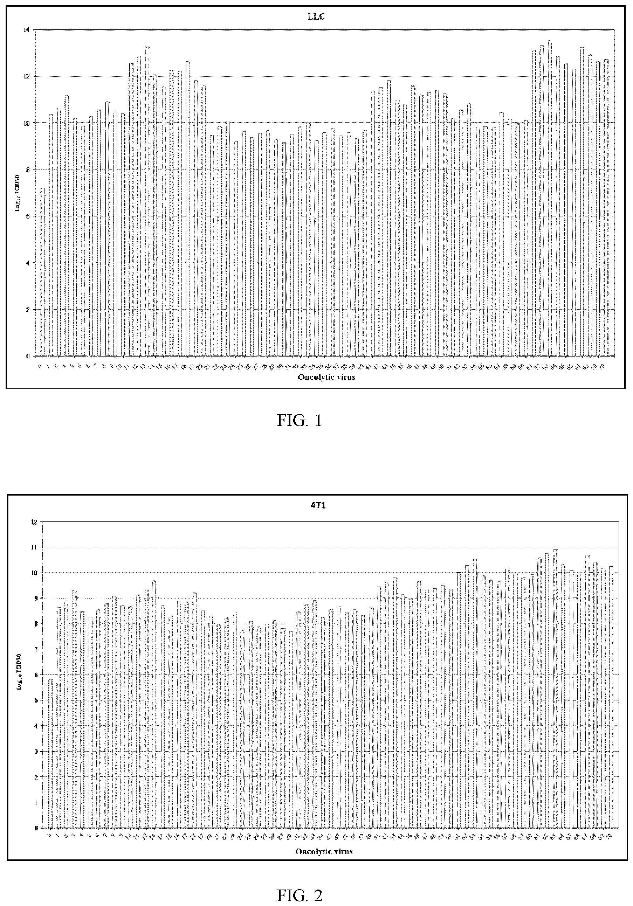

The test results are shown in FIGS. 1-10. As shown in the figures, the number 0 on the horizontal axis represents the wild-type oncolytic virus; the numbers 1-105 on the horizontal axis represent the recombinant oncolytic viruses prepared in Preparation Examples 1-105, respectively; Log₁₀ TCID50 on the vertical axis represents the values of TCID50 calculated by the Karber method; the larger the value of Log₁₀ TCID50, the better the infective ability of a recombinant oncolytic virus to the cell; the smaller the value of Log₁₀ TCID50, the poorer the poorer ability of the recombinant oncolytic virus to the cell.

FIG. 1 shows the test result of the infective ability of the recombinant oncolytic viruses prepared in Preparation Examples 1-70 of the present application and a wild-type oncolytic virus to LLC cell.

FIG. 2 shows the test result of the infective ability of the recombinant oncolytic viruses prepared in Preparation Examples 1-70 of the present application and a wild-type oncolytic virus to 4T1 cell.

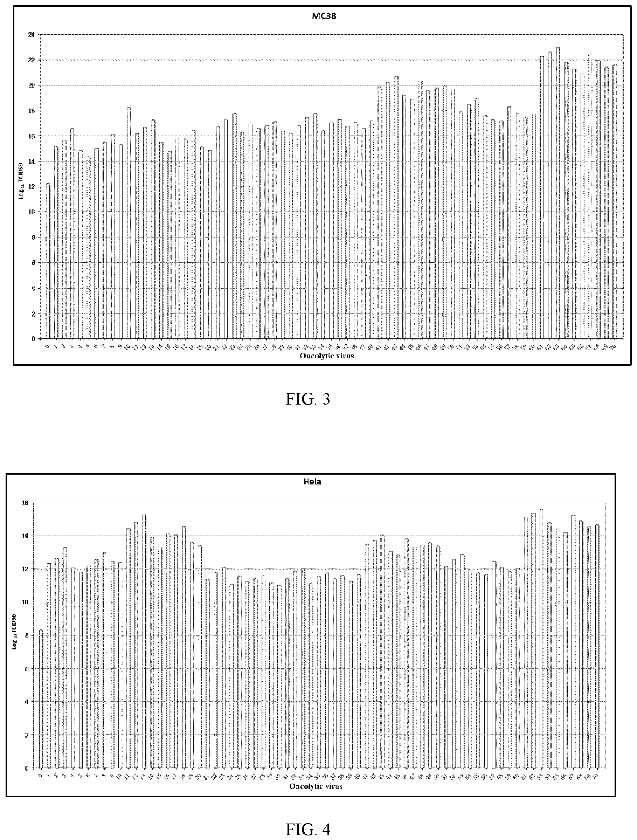

FIG. 3 shows the test result of the infective ability of the recombinant oncolytic viruses prepared in Preparation Examples 1-70 of the present application and a wild-type oncolytic virus to MC38 cell.

FIG. 4 shows the test result of the infective ability of the recombinant oncolytic viruses prepared in Preparation Examples 1-70 of the present application and a wild-type oncolytic virus to Hela cell.

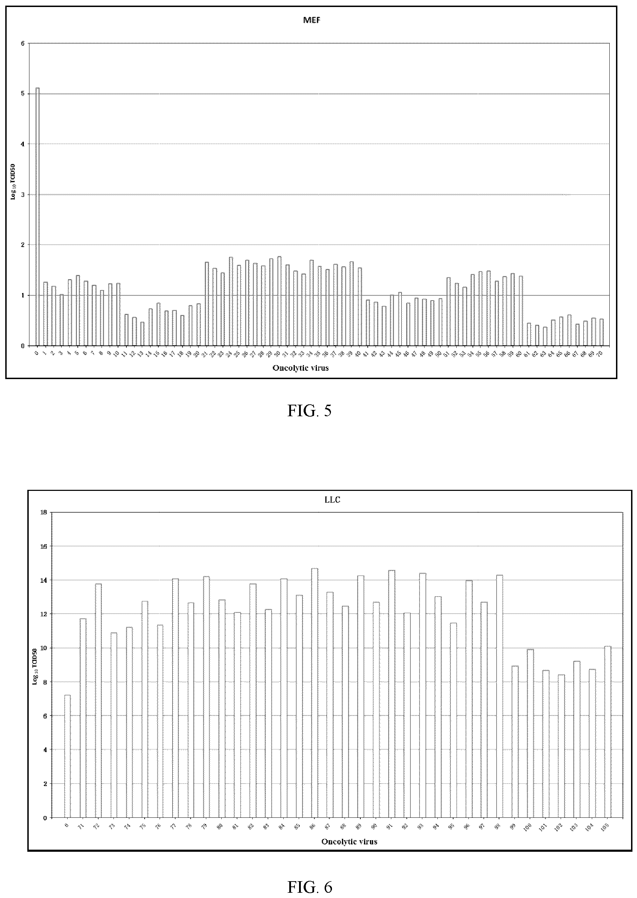

FIG. 5 shows the test result of the infective ability of the recombinant oncolytic viruses prepared in Preparation Examples 1-70 of the present application and a wild-type oncolytic virus to MEF cell.

FIG. 6 shows the test result of the infective ability of the recombinant oncolytic viruses prepared in Preparation Examples 71-105 of the present application and a wild-type oncolytic virus to LLC cell.

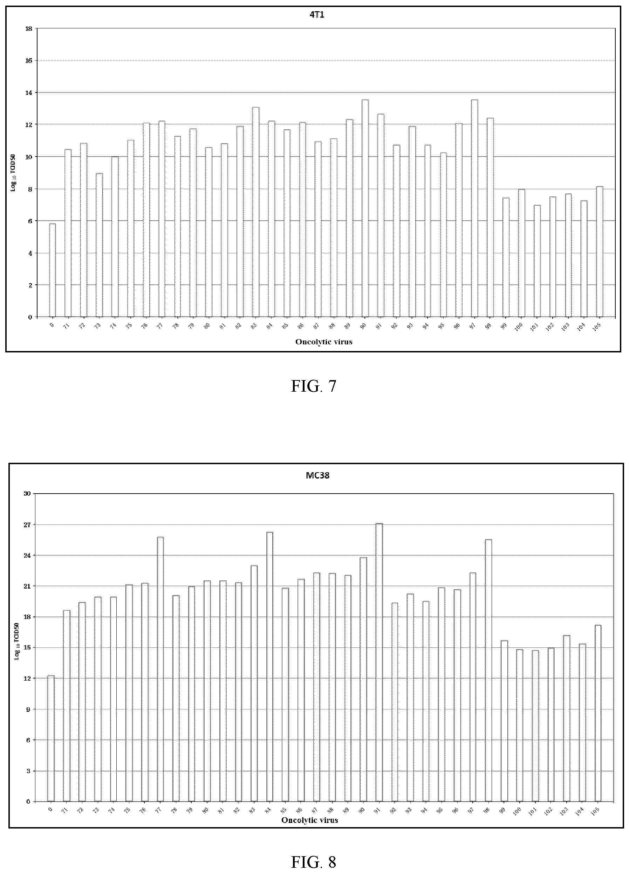

FIG. 7 shows the test result of the infective ability of the recombinant oncolytic viruses prepared in Preparation Examples 71-105 of the present application and a wild-type oncolytic virus to 4T1 cell.

FIG. 8 shows the test result of the infective ability of the recombinant oncolytic viruses prepared in Preparation Examples 71-105 of the present application and a wild-type oncolytic virus to MC38 cell.

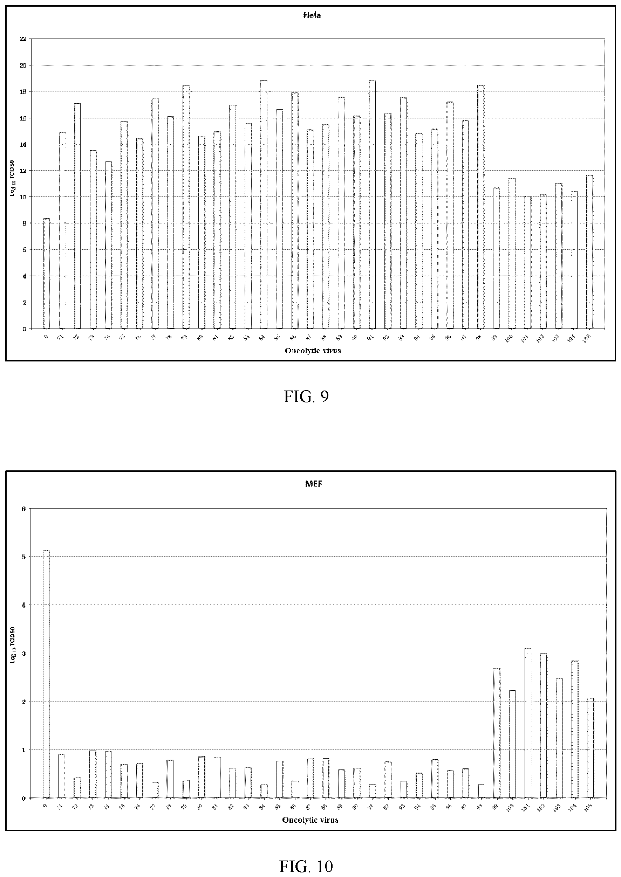

FIG. 9 shows the test result of the infective ability of the recombinant oncolytic viruses prepared in Preparation Examples 71-105 of the present application and a wild-type oncolytic virus to Hela cell.

FIG. 10 shows the test result of the infective ability of the recombinant oncolytic viruses prepared in Preparation Examples 71-105 of the present application and a wild-type oncolytic virus to MEF cell.

It can be seen from the above figures that the recombinant oncolytic viruses prepared in Preparation Examples 1-98 of the present application have good infective ability to the LLC cell, the 4T1 cell, the MC38 cell and the Hela cell, and are all superior to the recombinant oncolytic viruses prepared in Preparation Examples 99-105 using wild-type oncolytic viruses combined with cytokines in the infective ability to the LLC cell, the 4T1 cell, the MC38 cell and the Hela cell. And especially the infective ability of the recombinant oncolytic viruses provided in Preparation Examples 61-70 to the LLC cell, the 4T1 cell, the MC38 cell and the Hela cell is significantly better than other preparation examples. Moreover, the prepared recombinant oncolytic viruses as described above all have poor infective ability to the MEF cell, indicating that the recombinant oncolytic viruses prepared in the present application can be well used for infecting tumor and cancer cells without damaging normal cells, thus achieving broad application prospects.

### Example 2

In this example, a killing ability of the recombinant oncolytic viruses prepared in Preparation Examples 1-105 and the wild-type oncolytic virus to different cells in vitro were respectively detected.

The detection method was an MTT detection method, that is, 200 pfu of each of the recombinant oncolytic viruses prepared in Preparation Examples 1-105 and 200 pfu of the wild-type oncolytic virus were added to culture solutions of different cells, and a cell activity was detected using the MTT detection method after 24 hour.

The cells tested included: LLC cell, 4T1 cell, MC38 cell, Hela cell, and MEF cell. Specifically, the detection method included the following steps.
(1) 100 mL of Vero (LLC/4T1/MC38/Hela/MEF) cell suspension was added to each well of a 96-well plate, with 1×10⁴ cells/well; the 96-well culture plate was cultured for 16 hours under the environmental condition of 37°C and 5% of CO₂;
(2) Each of the recombinant oncolytic viruses prepared in the preparation examples was diluted to an MOI (multiplicity of infection) of 0.001, 0.01, 0.1, and 1.0, and the diluted recombinant oncolytic viruses were inoculated into the 96-well plate from step (1), 4 wells for each dilution gradient, 100 µl/well; and the 96-well plate was then cultured for 40 hours under the environmental condition of 37°C and 5% of CO₂;
(3) Cell supernatants were removed from the 96-well culture plate from step (2), fresh culture medium and MTT solution were then added to the 96-well plate with 20 µL/well; and the 96-well plate was then cultured for 4 hours under the environmental condition of 37°C and 5% of CO₂;
(4) The 96-well plate was centrifuged at room temperature, 2500 rpm/min for 5 min and the supernatants were gently removed with a 1 mL disposable sterile syringe; then, DMSO was added to the wells of the 96-well plate with 100 µl/well; and the 96-well plate was then placed at 37 °C for 10 min. The 96-well plate was shaken for 2 min and the OD value of each well in the 96-well plate was measured by ELIASA at a wavelength of 570 nm or 490 nm.

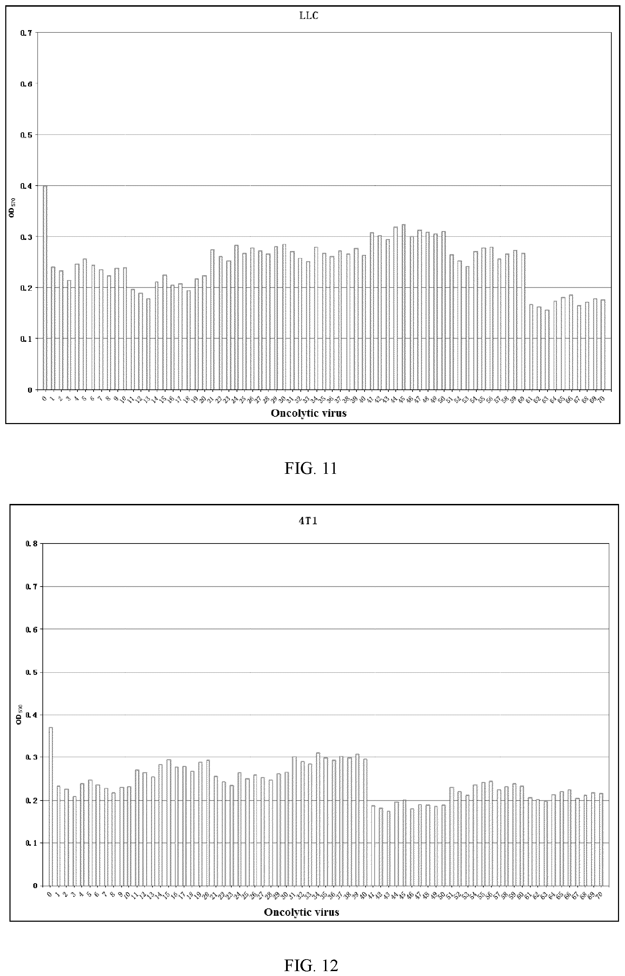

Test results are shown in FIGS. 11-20. As shown in the figures, the number 0 on the horizontal axis represents the wild-type oncolytic virus; the numbers 1-105 on the horizontal axis represent the recombinant oncolytic viruses prepared in Preparation Examples 1-105, respectively; OD₅₇₀ on the vertical axis represents the OD values of the cells. The larger the value of OD₅₇₀, the poorer the killing ability of a recombinant oncolytic virus to the cell; the smaller the value of OD₅₇₀, the better the killing ability of the recombinant oncolytic virus to the cell.

FIG. 11 shows the test result of the killing ability of the recombinant oncolytic viruses prepared in Preparation Examples 1-70 of the present application and a wild-type oncolytic virus to LLC cell in vitro.

FIG. 12 shows the test result of the killing ability of the recombinant oncolytic viruses prepared in Preparation Examples 1-70 of the present application and a wild-type oncolytic virus to 4T1 cell in vitro.

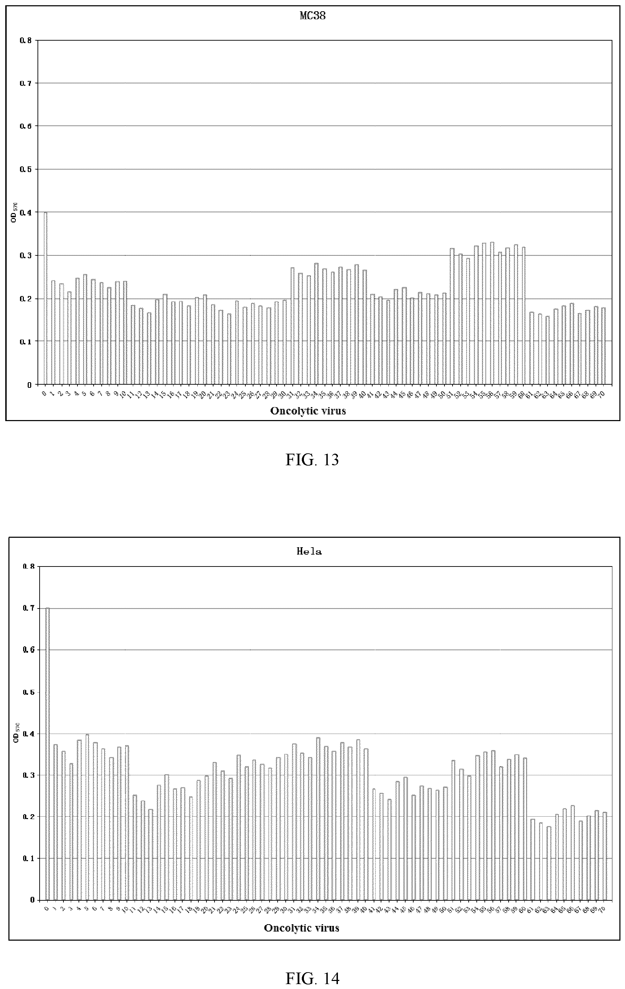

FIG. 13 shows the test result of the killing ability of the recombinant oncolytic viruses prepared in Preparation Examples 1-70 of the present application and a wild-type oncolytic virus to MC38 cell in vitro.

FIG. 14 shows the test result of the killing ability of the recombinant oncolytic viruses prepared in Preparation Examples 1-70 of the present application and a wild-type oncolytic virus to Hela cell in vitro.

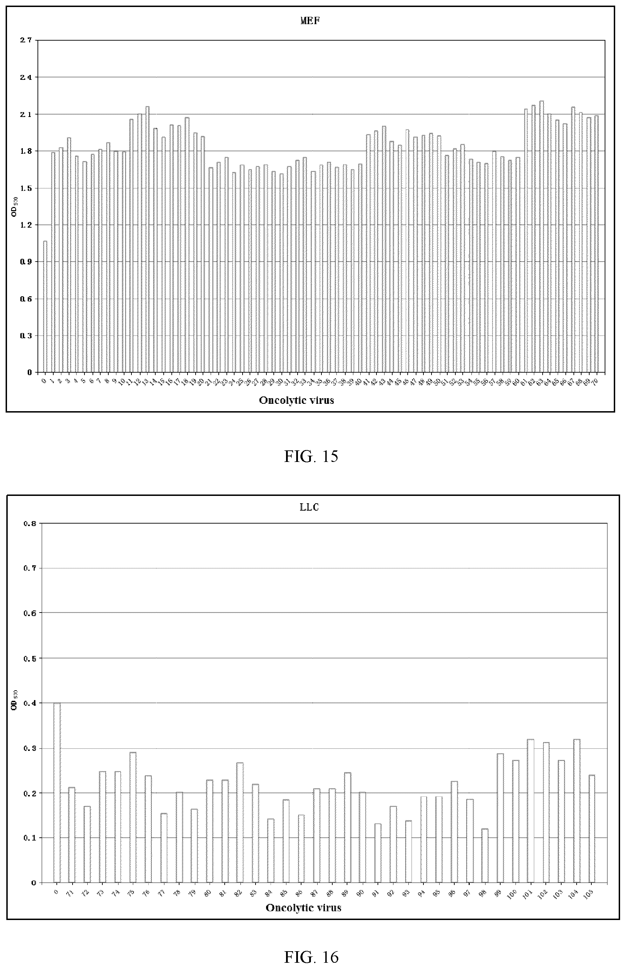

FIG. 15 shows the test result of the killing ability of the recombinant oncolytic viruses prepared in Preparation Examples 1-70 of the present application and a wild-type oncolytic virus to MEF cell in vitro.

FIG. 16 shows the test result of the killing ability of the recombinant oncolytic viruses prepared in Preparation Examples 71-105 of the present application and a wild-type oncolytic virus to LLC cell in vitro.

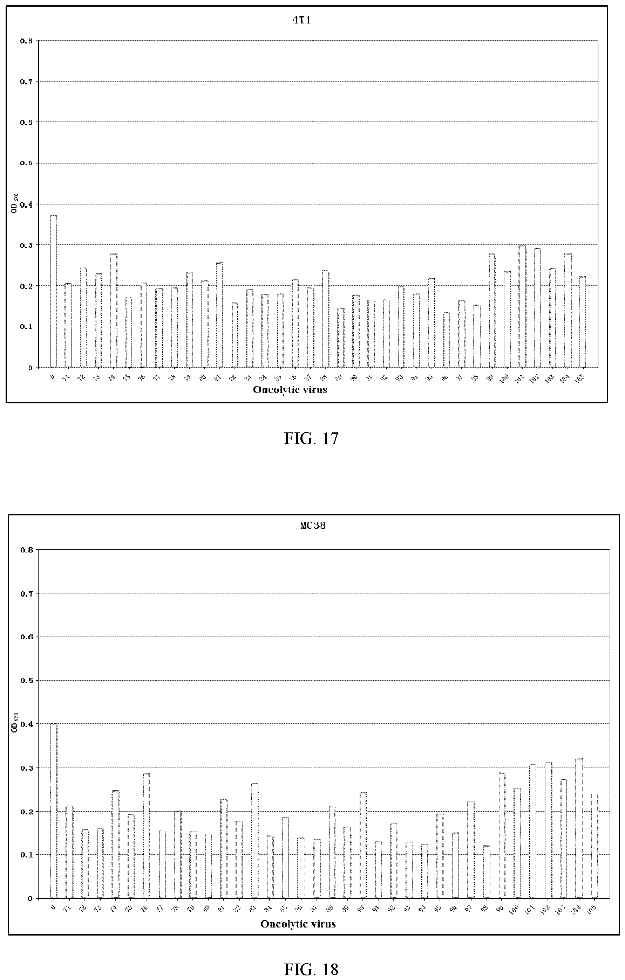

FIG. 17 shows the test result of the killing ability of the recombinant oncolytic viruses prepared in Preparation Examples 71-105 of the present application and a wild-type oncolytic virus to 4T1 cell in vitro.

FIG. 18 shows the test result of the killing ability of the recombinant oncolytic viruses prepared in Preparation Examples 71-105 of the present application and a wild-type oncolytic virus to MC38 cell in vitro.

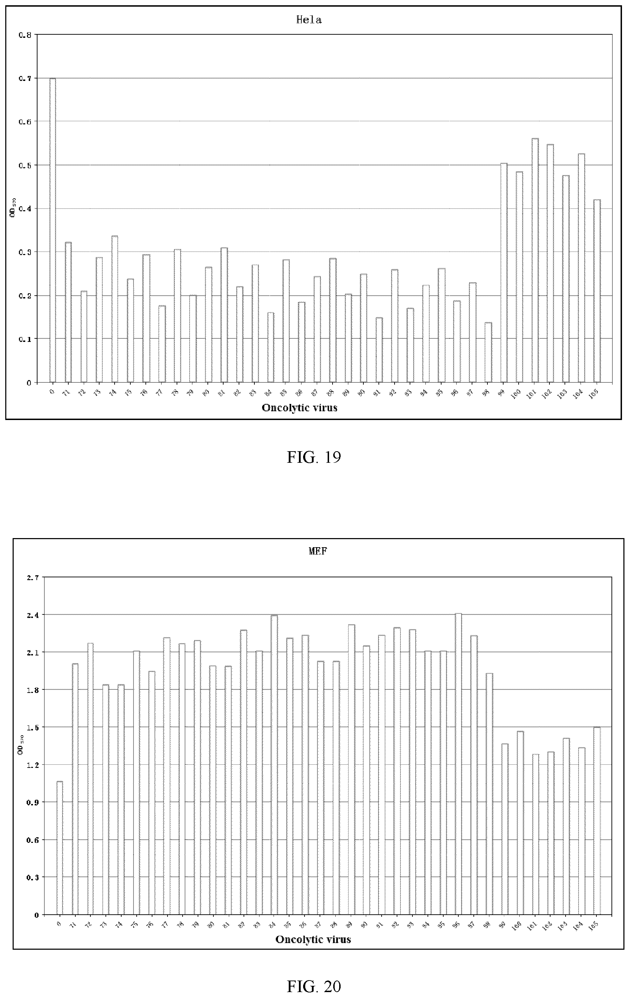

FIG. 19 shows the test result of the killing ability of the recombinant oncolytic viruses prepared in Preparation Examples 71-105 of the present application and a wild-type oncolytic virus to Hela cell in vitro.

FIG. 20 shows the test result of the killing ability of the recombinant oncolytic viruses prepared in Preparation Examples 71-105 of the present application and a wild-type oncolytic virus to MEF cell in vitro.

It can be seen from the above figures that the recombinant oncolytic viruses prepared in Preparation Examples 1-98 of the present application have better killing ability to the LLC cell, the 4T1 cell, the MC38 cell and the Hela cell in vitro, and are all superior to the killing ability of the recombinant oncolytic viruses prepared in Preparation Examples 99-105 using wild-type oncolytic viruses combined with cytokines to the LLC cell, the 4T1 cell, the MC38 cell and the Hela cell in vitro. And especially the recombinant oncolytic viruses provided in Preparation Examples 61-70 have better killing ability to the LLC cell, the 4T1 cell, the MC38 cell and the Hela cell in vitro than the wild-type oncolytic virus. Moreover, the recombinant oncolytic viruses prepared almost have no effect on MEF cells, indicating that the recombinant oncolytic viruses prepared herein can be well used for damaging and killing abnormal cells such as tumor and cancer cells, without causing damage to normal cells.

Although the wild-type oncolytic virus has better killing ability to the LLC cell, the MC38 cell and the 4T1 cell in vitro, while damaging and killing these cells, the wild-type oncolytic virus may also damage and kill the MEF cell to a large extent, thereby limiting the clinical application of the wild-type oncolytic virus. Therefore, the modification of the wild-type oncolytic virus in the present application not only ensures the safety of oncolytic viruses to normal cells, but also ensures the killing ability of oncolytic viruses to tumor and cancer cells, thereby achieving broad clinical application prospects.

### Example 3

In this example, the expression of IFN-β induced by the recombinant oncolytic viruses prepared in Preparation Examples 1-105 and the wild-type oncolytic virus in different cells was detected.

The test indicator was the expression of the gene IFN-β in different cells. The gene IFN-β is a soluble glycoprotein gene produced by cells and having a wide range of antiviral, anti-tumor and immunomodulatory effects. The expression of the gene IFN-β can be used to determine the ability of cells to eliminate a recombinant oncolytic virus: when the expression of the gene IFN-β is high, it indicates that the recombiant oncolytic virus is easy to eliminate in cells; when the expression of the gene IFN-β is low, it indicates that the recombiant oncolytic virus is not easy to eliminate in cells.

The cells tested included: LLC cell, 4T1 cell, MC38 cell, Hela cell, and MEF cell.

Specifically, the detection method included the following steps.
(1) 100 µL of Vero (LLC/4T1/MC38/Hela/MEF) cell suspension was added to each well of a 96-well plate, with 1×10⁴ cells/well; the 96-well culture plate was then cultured for 16 hours under an environmental condition of 37°C and 5% of CO₂;
(2) Each of the recombinant oncolytic viruses prepared in the preparation examples was diluted to an MOI (multiplicity of infection) of 0.001, 0.01, 0.1, and 1.0, and the diluted recombinant oncolytic viruses were inoculated into the 96-well plate from step (1), 4 wells for each dilution gradient, 100 µl/well; and the 96-well plate was then cultured for 40 hours under the environmental condition of 37°C and 5% of CO₂;
(3) Each group of cells obtained by culture in step (2) was broken, and total RNA was extracted from each cell using TRIzol (Invitrogen), reverse transcribed into cDNA by using a reverse transcription kit (PrimeScript RT Reagent Kit with DNA Eraser (Takara)), and stained with a dye (LightCycler 480SYBR Green I Master (Roche)), and the Ct value of each gene was detected on a LightCycler 480 quantitative PCR instrument. The relative expression level of the target gene IFN-β was calculated according to a ΔΔCt method.

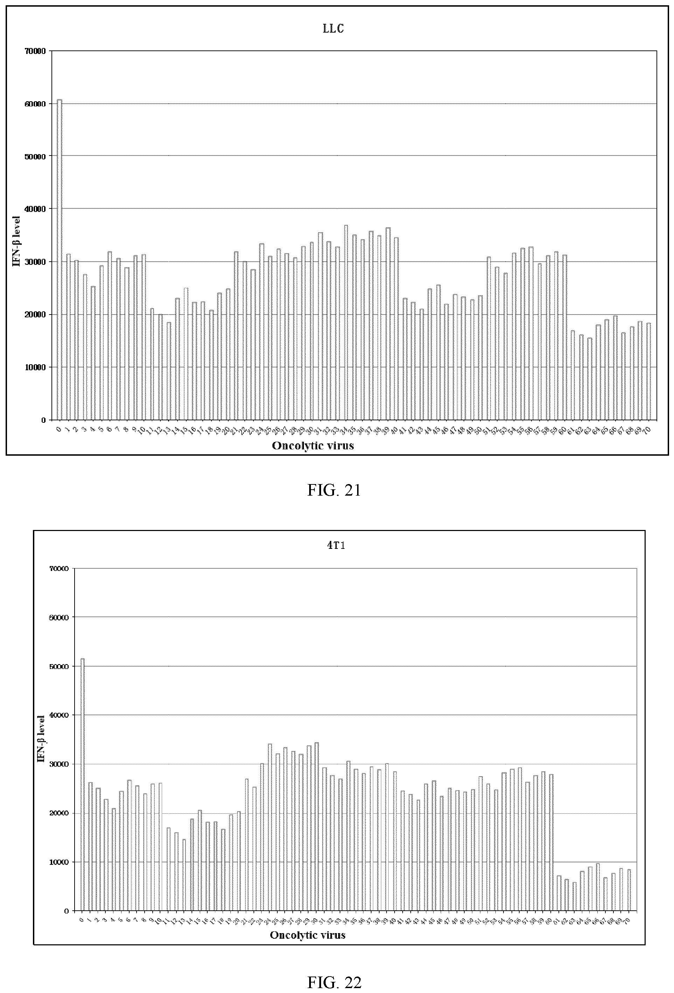

Test results are shown in FIGS. 21-30. As shown in the figures, the number 0 on the horizontal axis represents the wild-type oncolytic virus; the numbers 1-105 on the horizontal axis represent the recombinant oncolytic viruses prepared in Preparation Examples 1-105, respectively; the IFN-β level on the vertical axis represents the expression level of the IFN-β gene; the larger the value of the IFN-β level, the weaker the reproduction ability of a recombinant oncolytic virus in the cell and the easier of the recombinant oncolytic virus to eliminate in the cell; the smaller the value of the IFN-β level, the stronger the reproduction ability of the recombinant oncolytic virus in the cell, and the more difficult of the recombinant oncolytic virus to eliminate in the cell.

FIG. 21 shows the expression of IFN-β induced by the recombinant oncolytic viruses prepared in Preparation Example 1-70 of the present application and a wild-type oncolytic virus in LLC cell.

FIG. 22 shows the expression of IFN-β induced by the recombinant oncolytic viruses prepared in Preparation Example 1-70 of the present application and a wild-type oncolytic virus in 4T1 cell.

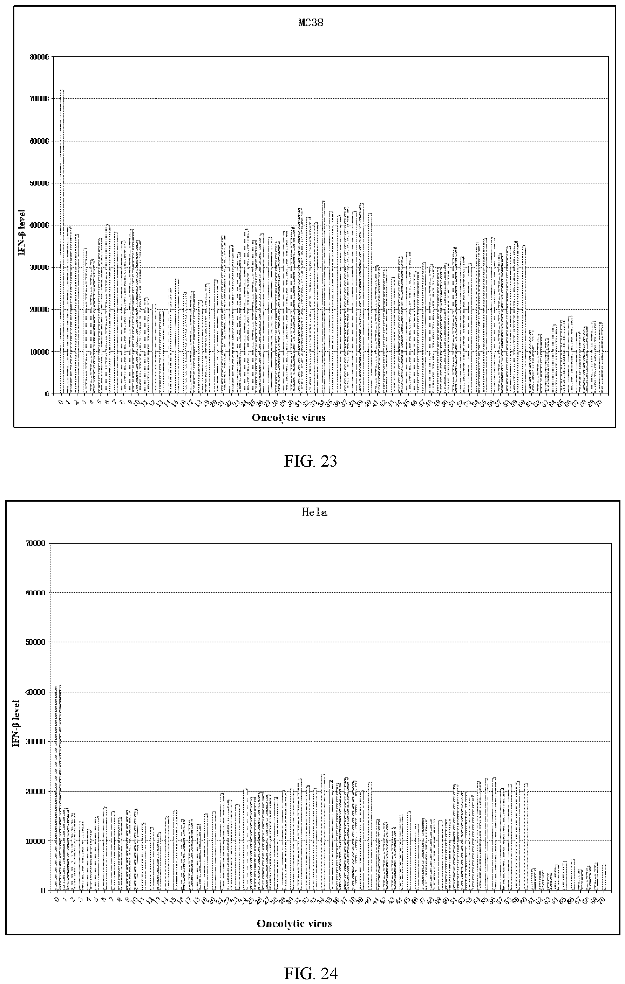

FIG. 23 shows the expression of IFN-β induced by the recombinant oncolytic viruses prepared in Preparation Example 1-70 of the present application and a wild-type oncolytic virus in MC38 cell.

FIG. 24 shows the expression of IFN-β induced by the recombinant oncolytic viruses prepared in Preparation Example 1-70 of the present application and a wild-type oncolytic virus in Hela cell.

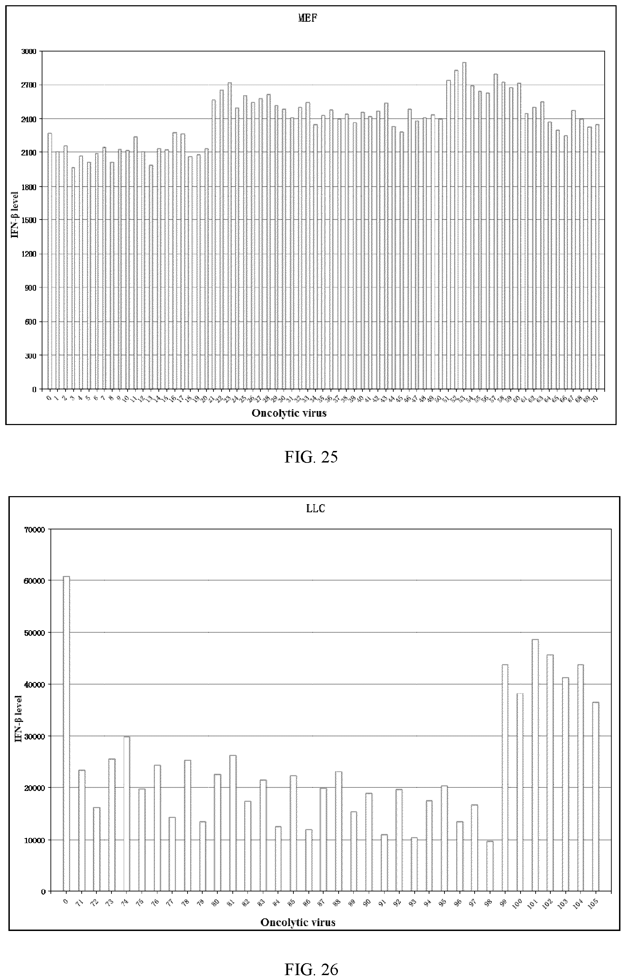

FIG. 25 shows the expression of IFN-β induced by the recombinant oncolytic viruses prepared in Preparation Example 1-70 of the present application and a wild-type oncolytic virus in MEF cell.

FIG. 26 shows the expression of IFN-β induced by the recombinant oncolytic viruses prepared in Preparation Example 71-105 of the present application and a wild-type oncolytic virus in LLC cell.

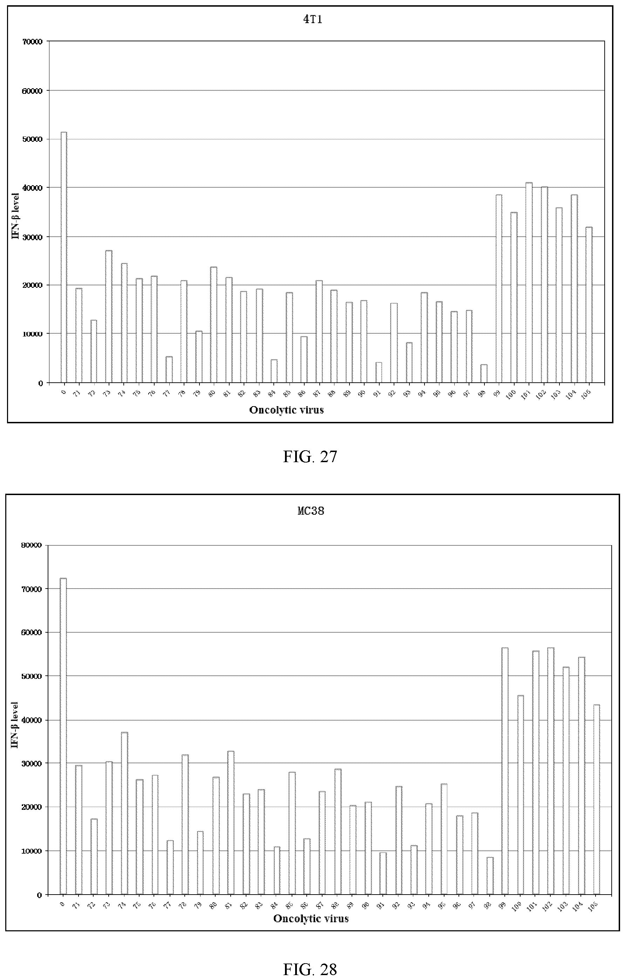

FIG. 27 shows the expression of IFN-β induced by the recombinant oncolytic viruses prepared in Preparation Example 71-105 of the present application and a wild-type oncolytic virus in 4T1 cell.

FIG. 28 shows the expression of IFN-β induced by the recombinant oncolytic viruses prepared in Preparation Example 71-105 of the present application and a wild-type oncolytic virus in MC38 cell.

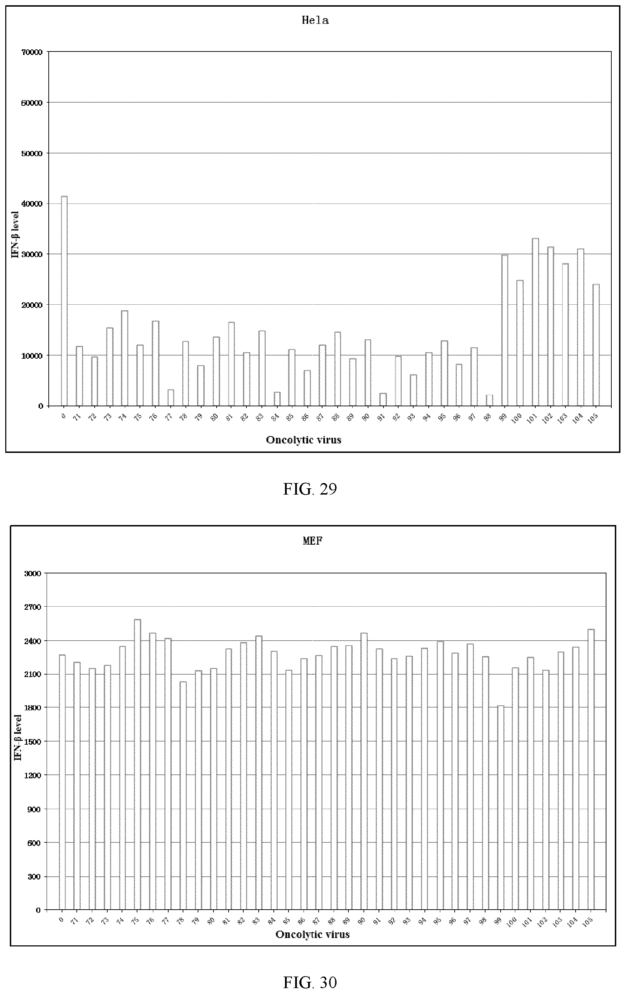

FIG. 29 shows the expression of IFN-β induced by the recombinant oncolytic viruses prepared in Preparation Example 71-105 of the present application and a wild-type oncolytic virus in Hela cell.

FIG. 30 shows the expression of IFN-β induced by the recombinant oncolytic viruses prepared in Preparation Example 71-105 of the present application and a wild-type oncolytic virus in MEF cell.

It can be seen from the above figures that the recombinant oncolytic viruses prepared in Preparation Examples 1-98 of the present application and the wild-type virus have good reproduction ability in the LLC cell, the 4T1 cell, the MC38 cell and the Hela cell and are difficultly eliminated, and are all superior to the reproduction ability of the recombinant oncolytic viruses prepared in Preparation Examples 99-105 using wild-type oncolytic viruses combined with cytokines in the LLC cell, the 4T1 cell, the MC38 cell and the Hela cell. And especially the recombinant oncolytic viruses provided in Preparation Examples 61-70 are more difficultly eliminated from the LLC cell, the 4T1 cell, the MC38 cell and the Hela cell, thereby further ensuring that these oncolytic viruses can better exert their ability to infect and kill the LLC cell, the 4T1 cell, the MC38 cell and the Hela cell. Moreover, the recombinant oncolytic viruses of the present application are more easier to eliminated in the MEF cell, thereby further ensuring the safety of the MEF cell and improving the safety of the oncolytic viruses.

The specific examples are merely an explanation of the present application and not for limiting the present application. Those skilled in the art may make modifications, without creative contribution, to the examples as needed after reading this specification. Any of the modifications made within the scope of the claims of the present application shall be protected by the Patent Law.

## Claims

1. A recombinant oncolytic virus, **characterized by** comprising an M protein and a cytokine encoded by an exogenous gene, wherein the M protein comprises the following site mutations compared to an amino acid sequence as shown in SEQ ID NO 1: mutating of methionine at position 51 into arginine (M51R); mutating of valine at position 221 into phenylalanine (V221F); and mutating of serine at position 226 into arginine (S226R).

2. The recombinant oncolytic virus according to claim 1, **characterized in that**, the M protein further comprises one or more of the following site mutations: mutating of asparagine at position 32 into serine (N32S); and/or mutating of asparagine at position 49 into aspartic acid (N49D); and/or mutating of histidine at position 54 into tyrosine (H54Y); and/or mutating of valine at position 225 into isoleucine (V225I).

3. The recombinant oncolytic virus according to claim 1 or 2, **characterized in that**, the M protein further comprises one or more of the following site mutations: knocking out of leucine at position 111; or, mutating of leucine at position 111 into alanine (L111A).

4. The recombinant oncolytic virus according to claim 2 or 3, **characterized in that**, the M protein further comprises one or more of the following site mutations: mutating of glycine at position 21 into alanine (G21E); and/or mutating of methionine at position 33 into alanine (M33A); and/or mutating of alanine at position 133 into threonine (A133T).

5. The recombinant oncolytic virus according to any one of claims 1 to 4, **characterized in that**, the site mutation of the M protein comprises site mutations in any one of the following groups:
1) M51R, V221F and S226R;
2) N32S, N49D, M51R, H54Y, V221F, V225I and S226R;
3) N32S, N49D, M51R, H54Y, knocking out of leucine at position 111, V221F, V225I and S226R;
4) N32S, N49D, M51R, H54Y, L111A, V221F, V225I and S226R;
5) G21E, N32S, N49D, M51R, H54Y, V221F, V225I and S226R;
6) G21E, N32S, M33A, N49D, M51R, H54Y, V221F, V225I and S226R;
7) G21E, N32S, M33A, N49D, M51R, H54Y, A133T, V221F, V225I and S226R;
8) N32S, M33A, N49D, M51R, H54Y, V221F, V225I and S226R;
9) N32S, M33A, N49D, M51R, H54Y, A133T, V221F, V225I and S226R; and
10) N32S, N49D, M51R, H54Y, A133T, V221F, V225I and S226R.

6. The recombinant oncolytic virus according to claim 5, **characterized in that**, the M protein comprises an amino acid sequence as shown in SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10 or SEQ ID NO 11.

7. The recombinant oncolytic virus according to any one of claims 1 to 6, **characterized in that**, the cytokine is selected from interleukins (IL), interferons (IFN), tumor necrosis factors (TNF), colony stimulating factors (CSF), transforming growth factor-β family (TGF-β family) and chemokine family.

8. The recombinant oncolytic virus according to claim 7, **characterized in that**, the cytokine is at least one selected from a group consisting of: GM-CSF, G-CSF, M-CSF, IL-1, IL-2, IL-4, IL-5, IL-6, IL-9, IL-10, IL-12, IL-13, IL-15, IL-17, IL-18, IL-23, IL-27, IFN-a, IFN-β, IFN-γ, IFN-β, TGF-β and TNF-α.

9. The recombinant oncolytic virus according to claim 8, **characterized in that**, the cytokine is at least one selected from a group consisting of: GM-CSF, IL-2, IL-12, IL-15, IL-18, IFN-β and TNF-α.

10. A recombinant oncolytic virus, **characterized by** comprising the M protein according to any one of claims 1 to 9; the recombinant oncolytic virus further comprises a G protein, wherein the G protein comprises one or more of the following site mutations compared to an amino acid sequence shown in SEQ ID NO 12: mutating of valine at position 53 into isoleucine (V531); and/or, mutating of alanine at position 141 into valine (A141V); and/or, mutating of aspartic acid at position 172 into tyrosine (D172Y); and/or, mutating of lysine at position 217 into glutamic acid (K217E); and/or, mutating of aspartic acid at position 232 into glycine and/or, mutating of valine at position 331 to alanine (V331A); and/or, mutating of valine at position 371 into glutamic acid (V371E); and/or, mutating of glycine at position 436 into aspartic acid (G436D); and/or, mutating of threonine at position 438 into serine (T438S); and/or, mutating of phenylalanine at position 453 into leucine (F453L); and/or, mutating of threonine at position 471 into isoleucine (T471I); and/or, mutating of tyrosine at position 487 into histidine (Y487H).

11. The recombinant oncolytic virus according to claim 10, **characterized in that**, the G protein comprises an amino acid sequence as shown in SEQ ID NO 13.

12. A recombinant oncolytic virus, **characterized by** comprising the M protein according to any one of claims 1 to 9, or the M protein and G protein according to claim 10 or 11; the recombinant oncolytic virus further comprises an N protein, wherein the N protein comprises one or more of the following site mutations compared to an amino acid sequence as shown in SEQ ID NO 14: mutating of isoleucine at position 14 into valine (I14V); and/or, mutating of arginine at position 155 into lysine (R155K); and/or, mutating of serine at position 353 into asparagine (S353N).

13. The recombinant oncolytic virus according to claim 12, **characterized in that**, the N protein comprises an amino acid sequence as shown in SEQ ID NO 15.

14. A recombinant oncolytic virus, **characterized by** comprising the M protein according to any one of claims 1 to 9; or the M protein and G protein according to claim 10 or 11; or the M protein, G protein and N protein according to claim 12 or 13; the recombinant oncolytic virus further comprises a P protein, wherein the P protein comprises one or more of the following site mutations compared to an amino acid sequence as shown in SEQ ID NO 16: mutating of arginine at position 50 into lysine (R50K); and/or mutating of valine at position 76 into alanine (V76A); and/or mutating of asparagine at position 99 into glutamic acid (D99E); and/or mutating of leucine at position 126 into serine (L126S); and/or mutating of leucine at position 140 into serine (L140S); and/or mutating of histidine at position 151 into tyrosine (H151Y); and/or mutating of isoleucine at position 168 into methionine (T168M); and/or mutating of lysine at position 170 into glutamic acid (K170E); and/or mutating of tyrosine at position 189 into serine (Y189S); and/or mutating of asparagine at position 237 into aspartic acid (N237D).

15. The recombinant oncolytic virus according to claim 14, **characterized in that**, the P protein comprises an amino acid sequence as shown in SEQ ID NO 17.

16. A recombinant oncolytic virus, **characterized by** comprising the M protein according to any one of claims 1 to 9; or the M protein and G protein according to claim 10 or 11; or the M protein, G protein and N protein according to claim 12 or 13; or the M protein, G protein, N protein and P protein according to claim 14 or 15; the recombinant oncolytic virus further comprises an L protein, wherein the L protein comprises one or more of the following site mutations compared to an amino acid sequence as shown in SEQ ID NO 18: mutating of serine at position 87 into proline (S87P); and/or, mutating of isoleucine at position 487 into threonine (I487T).

17. The recombinant oncolytic virus according to claim 16, **characterized in that**, the L protein comprises an amino acid sequence as shown in SEQ ID NO 19.

18. The recombinant oncolytic virus according to any one of claims 1 to 17, **characterized by** further comprising a rhabdovirus.

19. The recombinant oncolytic virus according to any one of claims 1 to 17, **characterized by** further comprising a Vesicular Stomatitis Virus (VSV).

20. The recombinant oncolytic virus according to any one of claims 1 to 17, **characterized by** further comprising an Indiana MuddSummer subtype strain of the VSV.

21. The recombinant oncolytic virus according to any one of claims 1 to 20, **characterized by** further comprising or expressing an exogenous target protein.

22. The recombinant oncolytic virus according to any one of claims 1 to 21, **characterized in that**, the recombinant oncolytic virus comprises a nucleic acid molecule, wherein the nucleic acid molecule comprises a nucleic acid sequence encoding the M protein with the site mutation(s), and/or a nucleic acid sequence encoding the G protein with the site mutation(s), and/or a nucleic acid sequence encoding the N protein with the site mutation(s), and/or a nucleic acid sequence encoding the P protein with the site mutation(s), and/or a nucleic acid sequence encoding the L protein with the site mutation(s), and a nucleic acid sequence encoding the cytokine.

23. The recombinant oncolytic virus according to claim 22, **characterized in that**, in the nucleic acid molecule, the nucleic acid sequence encoding the cytokine is located between the nucleic acid sequence encoding the G protein with the site mutation(s) and the nucleic acid sequence encoding the L protein with the site mutation(s).

24. The recombinant oncolytic virus according to claim 22, **characterized in that**, in the nucleic acid molecule, the nucleic acid sequence encoding the cytokine is located between the nucleic acid sequence encoding the M protein with the site mutation(s), the nucleic acid sequence encoding the N protein with the site mutation(s) or the nucleic acid sequence encoding the P protein with the site mutation(s) and the nucleic acid sequence encoding the L protein with the site mutation(s).

25. An expression vector of the recombinant oncolytic virus capable of expressing the recombinant oncolytic virus according to any one of claims 1 to 24.

26. A virus-producing cell capable of producing the recombinant oncolytic virus according to any one of claims 1 to 24.

27. A vaccine prepared by using the recombinant oncolytic virus according to any one of claims 1 to 24.

28. A pharmaceutical composition, **characterized by** comprising the recombinant oncolytic virus according to any one of claims 1 to 24 or the vaccine according to claim 27, and optionally a pharmaceutically acceptable carrier.

29. A method for preparing the recombinant oncolytic virus according to any one of claims 1 to 24, the expression vector of the recombinant oncolytic virus according to claim 25, the virus-producing cell according to claim 26, the vaccine according to claim 27 or the pharmaceutical composition according to claim 28.

30. Use of the recombinant oncolytic virus according to any one of claims 1 to 24, the expression vector of the recombinant oncolytic virus according to claim 25, the virus-producing cell according to claim 26, the vaccine according to claim 27 or the pharmaceutical composition according to claim 28 in preparing a medicine for preventing and/or treating a disease and/or disorder.

31. The use according to claim 30, **characterized in that**, the recombinant oncolytic virus, the expression vector of the recombinant oncolytic virus, the virus-producing cell, the vaccine and/or the pharmaceutical composition are used in a method for sustained killing of an abnormally proliferative cell.

32. The use according to claim 31, **characterized in that**, the abnormally proliferative cell is selected from a group consisting of: a tumor cell and a cell associated with a tumor tissue.

33. Use of the recombinant oncolytic virus according to any one of claims 1 to 24, the vaccine according to claim 27 or the pharmaceutical composition according to claim 28 in preparing a medicine for treating a tumor.

34. The use according to claim 33, **characterized in that**, the tumor comprises a solid tumor or a hematological tumor.

35. The use according to claim 33, **characterized in that**, the tumor comprises but is not limited to acute lymphoblastic leukemia, acute B-cell leukemia, chronic non-lymphocytic leukemia, non-Hodgkin's lymphoma, anal cancer, astrocytoma, basal cell carcinoma, bile duct cancer, bladder cancer, mastocarcinoma, breast cancer, cervical cancer, chronic myeloproliferative tumors, colorectal cancer, endometrial cancer, ependymoma, esophageal cancer, diffuse large B-cell lymphoma, sensorineuroblastoma, Ewing's sarcoma, fallopian tube cancer, gallbladder cancer, gastric cancer, gastrointestinal carcinoid tumors, hepatocellular carcinoma, hypopharyngeal cancer, Kaposi sarcoma, kidney cancer, Langerhans cell hyperplasia, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumors, pharyngeal cancer, pituitary tumors, prostate cancer, rectal cancer, renal cell carcinoma, retinoblastoma, skin cancer, small cell lung cancer, small intestine cancer, squamous neck cancer, testicular cancer, thymoma, thyroid cancer, uterine cancer, vaginal cancer and vascular tumors.
